# EUROPÄISCHE PATENTSCHRIFT

(11) **EP 3 995 162 B1**
(45) Veröffentlichungstag und Bekanntmachung des Hinweises auf die Patenterteilung: **29.11.2023**
(21) Anmeldenummer: 21157125.2
(22) Anmeldetag: 15.02.2021
(51) Int. Cl.: A61M 1/02, A61J 1/10, A61J 1/16, A61M 5/44, B01L 7/00

(54) **AUFTAUVORRICHTUNG ZUM AUFTAUEN EINES SUBSTRATS SOWIE VERFAHREN ZUM AUFTAUEN EINES SUBSTRATS**
THAWING DEVICE FOR THAWING A SUBSTRATE AND METHOD FOR THAWING A SUBSTRATE
DISPOSITIF DE DÉGIVRAGE PERMETTANT DE DÉGIVRER UN SUBSTRAT, AINSI QUE PROCÉDÉ DE DÉGIVRAGE D'UN SUBSTRAT

(30) Priorität: 06.11.2020 DE 102020129292
(43) Veröffentlichungstag der Anmeldung: 11.05.2022
(73) Patentinhaber: Schmidt, Günther, 53123 Bonn (DE); Agentur Leven GmbH, 50858 Köln (DE)
(72) Erfinder: Schmidt, Günther, 53123 Bonn (DE)
(74) Vertreter: Hohendorf Kierdorf Patentanwälte PartGmbB

(56) Entgegenhaltungen:
- EP-A1- 2 839 822
- US-A- 4 473 739
- US-A- 4 742 202
- US-A- 5 261 255
- US-A1- 2006 032 607
- US-A1- 2019 336 660

## Beschreibung

Die vorliegende Erfindung betrifft eine Auftauvorrichtung zum Auftauen eines in gefrorenem Aggregatszustand befindlichen Substrats, insbesondere Blutplasma, wobei das Substrat in einem Behältnis, insbesondere einem Beutel, enthalten ist. Die Auftauvorrichtung umfasst eine Aufnahmeeinheit zur Anordnung des das Substrat enthaltenden Behältnisses, wobei die Aufnahmeeinheit eine Heizeinrichtung zur Bereitstellung von zum Auftauen des Substrats benötigter Wärmeenergie aufweist. Ferner umfasst die Auftauvorrichtung eine erste Bewegungseinrichtung, die dazu eingerichtet ist, eine erste Bewegung auf die Aufnahmeeinheit sowie das darin aufgenommene Behältnis samt Substrat zu übertragen, wobei die erste Bewegung eine im Wesentlichen in einer Ebene ausgeführte Bewegung, insbesondere eine Linearbewegung, ist.

Weiterhin betrifft die Erfindung ein Verfahren zum Auftauen eines in gefrorenem Aggregatszustand befindlichen Substrats, insbesondere Blutplasma, wobei das Substrat in einem Behältnis, insbesondere einem Beutel, enthalten ist.

Aus dem Stand der Technik sind Auftauvorrichtungen zum Auftauen medizinischer Substrate wohlbekannt (z.B. US 2006/032607 A1, US 4 742 202 A, EP 2 839 822 A1, US 2019/336660 A1, US 4 473 739 A, US 5 261 255 A).

Beispielhaft sei zunächst auf die Publikation DE 195 48 826 A1 eingegangen, welche eine gattungsgemäße Auftauvorrichtung offenbart. Die dort beschriebene Problemstellung lässt sich - zumindest in Teilen - auf die vorliegende Erfindung übertragen, weshalb nachfolgend zunächst die in der DE 195 48 826 A1 bereits thematisierte beschriebene Problemstellung angeführt sei.

Wie dort beschrieben, ist es in der klinischen Praxis üblich, pharmazeutische Wirkstoffe, die von der Industrie in Standardkonzentrationen und Standardvolumina geliefert werden, mit Trägerlösungen auf patientenspezifische Konzentration zu verdünnen und zu verabreichen. Da diese individuell angesetzten Präparate oft in langfristigen Therapien eingesetzt werden, wird bei der Herstellung der patientenspezifischen Wirkstoffmischung ein entsprechend großes Gesamtvolumen angesetzt. Die für die Einzelverabreichungen vorgesehenen Teilvolumina des Gesamtvolumens müssen deshalb in Spritzen oder anderen Behältnissen abgefüllt und anschließend gefroren aufgehoben werden. Nur so können die in der Regel thermisch labilen Wirkstoffe über die geforderten langen Zeiträume konserviert werden.

Ein Problem dieses eingeführten und weit verbreiteten herkömmlichen Konservierungsverfahrens ist, dass für die Einzelproben jeweils eine relativ lange Auftauzeit vorzusehen ist. Dies ist vor allem auch insofern umständlich, als der Zeitpunkt der Verabreichung des nächsten Wirkstoffvolumens nicht exakt vorherbestimmt werden kann. Es wird für den einzelnen Patienten zwar ein Zeitplan für die Verabreichung der Wirkstoffvolumina erstellt, in der Regel wird dem Patienten jedoch zunächst eine Blutprobe entnommen, um erst nach deren Analyse zu entscheiden, ob und - falls ja - zu welchem genauen Zeitpunkt das nächste Wirkstoffteilvolumen appliziert werden kann. Diese Vorgehensweise führt daher oft zu Zeitplanverschiebungen und folglich zum Verlust der vorab aufgetauten Wirkstoffe.

Dieser Problematik wurde mit dem dort beschriebenen Schnellauftaugerät entgegengetreten.

Ebenfalls wurde bereits in der DE 195 48 826 A1 problematisiert, dass gefrorene Flüssigkeiten auf Basis von Mikrowellenauftaugeräten aufgetaut werden können, welche dabei durch Mikrowellenstrahlung bei Frequenzen zwischen 2,425 und 2,475 GHz erwärmt werden. Diese weisen jedoch den Nachteil auf, dass überwiegend die gefrorene Flüssigkeit aufgrund der Fokussierung der Strahlung in einem bestimmten Bereich der gefrorenen Flüssigkeit stark erwärmt wird, so dass eine gleichmäßige Erwärmung der gefrorenen Flüssigkeit verhindert ist. Die Erhitzung der gefrorenen Flüssigkeit nur in einem bestimmten Bereich kann zu einer Schädigung von Inhalts- oder Wirkstoffen der Flüssigkeit führen, so dass mit einer chemischen Modifikation der Inhalts- bzw. der Wirkstoffe zu rechnen ist. Nebst einer chemischen Modifikation der (aufgetauten) Arzneimittel kann - je nach Art des Substrats - auch eine Denaturierung von Impfstoffen, Proteinen oder Seren in den Infusions- und Injektionslösungen durch die Applikation von Mikrowellenstrahlung erfolgen.

Ein händisches Auftauen von in Behältnissen enthaltenen Substraten unter warmem, fließendem Wasser, kann zu einer Kontamination des Substrats durch Mikroorganismen führen, welche von außen Öffnungsspalte, undichte Stellen oder dergleichen in das Behältnis eindringen können. Auch eine Erwärmung mittels der Handfläche des Benutzers schließt eine solche Kontamination durch Mikroorganismen nicht aus. Zudem ist das Auftauen in den Händen eines Benutzers mit einem relativ großen Zeitaufwand verbunden. Doch es ist gerade in Krankenhäusern bei der täglichen Routinearbeit, Notfallstationen oder bei Unfällen vor Ort dringend erforderlich und lebenswichtig, medizinische Substrate rasch auftauen und applizieren zu können.

Die DE 195 48 826 A1 schlägt zur Umgehung dieser Probleme vor, das aufzutauende Substrat beim Heizen (über Heizelemente, keine Mikrowelleneinrichtung) zusätzlich durch eine Schüttelmechanik in Bewegung zu versetzen. Gleichwohl mit dieser Technik ein im Vergleich zu einem Mikrowellen-basierten Auftauvorgang Substratschonenderes Auftauen sowie und ein im Vergleich zu den beschriebenen händischen Methoden zügigeres Auftauen ermöglicht ist, reichen die Auftauzeiten für bestimmte medizinische Substrate bzw. Anwendungen nicht aus. Als ein solches Beispiel für ein medizinisches Substrat, bei welchem eine Lagerung in gefrorenem Aggregatzustand üblich und ein schnelles Auftauen unabdingbar ist, sei Blutplasma genannt. Blutplasma wird in der Regel bei -30 bis -40°C gelagert (konserviert), kann jedoch nur in flüssigem Aggregatzustand (also aufgetaut) verabreicht werden.

Blutplasma muss bei großen, komplizierten Operationen und insbesondere bei Notfällen schnell verfügbar und applizierbar sein, um massiven Blutverlusten von Patienten (z.B. Verunglückten eines Unfalls) entgegenzuwirken. Auch bei Transplantationen können derart hohe Blutverluste auftreten, die eine zügige Verfügbarkeit von Blutplasma erforderlich machen.

Da mit den herkömmlichen (z.B. aus der DE 195 48 826 A1) bekannten Geräten und Methoden Auftauzeiten von ca. 30 Minuten verwirklicht werden können, wird Blutplasma bei anstehenden Operationen häufig auf "Vorrat" aufgetaut, damit es im Bedarfsfall unmittelbar zur Verfügung steht. Nicht benötigtes Plasma wird nach der Operation entsorgt, was sich negativ auf die ohnehin knappen Lagerbestände von Blutplasma auswirkt.

Die Bereitstellung kürzerer Auftauzeiten für gefrorenes Blutplasma ist also von signifikantem Interesse zur Gewährleistung einer effizienten und lebensrettenden Patientenversorgung. Gleichzeitig ist dabei zu beachten, dass ein schonendes Auftauen des Blutplasmas gewährleistet wird.

Entsprechend liegt der vorliegenden Erfindung die Aufgabe zugrunde, eine Auftauvorrichtung und ein Verfahren zum Auftauen eines in gefrorenem Aggregatszustand befindlichen Substrats, insbesondere Blutplasma, vorzuschlagen, wodurch eine im Vergleich zu Stand geringere Auftauzeit ermöglicht sowie zugleich ein schonender Auftauvorgang ermöglicht wird.

Die vorgenannte Aufgabe wird mit einer Auftauvorrichtung mit den Merkmalen des Anspruchs 1 sowie einem Verfahren mit den Merkmalen des Anspruchs 15 gelöst.

Es ist darauf hinzuweisen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich. Auch können die im Zusammenhang der erfindungsgemäßen Auftauvorrichtung beschriebenen Merkmale vorteilhafte Ausgestaltungen des erfindungsgemäßen Verfahrens sein und umgekehrt.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des erfindungsgemäßen Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Wie erwähnt, betrifft die vorliegende Erfindung zunächst Auftauvorrichtung zum Auftauen eines in gefrorenem Aggregatszustand befindlichen Substrats, insbesondere Blutplasma, wobei das Substrat in einem Behältnis, insbesondere einem Beutel, enthalten ist, die Auftauvorrichtung umfassend
a. eine Aufnahmeeinheit zur Anordnung des das Substrat enthaltenden Behältnisses, wobei die Aufnahmeeinheit eine Heizeinrichtung zur Bereitstellung von zum Auftauen des Substrats benötigter Wärmeenergie aufweist;
b. eine erste Bewegungseinrichtung, die dazu eingerichtet ist, eine erste Bewegung auf die Aufnahmeeinheit sowie das darin aufgenommene Behältnis samt Substrat zu übertragen, wobei die erste Bewegung eine im Wesentlichen in einer Ebene ausgeführte Bewegung, insbesondere eine Linearbewegung, ist.

Die Auftauvorrichtung zeichnet sich erfindungsgemäß aus durch c. eine zweite Bewegungseinrichtung, die dazu eingerichtet ist, eine zweite Bewegung auf die Aufnahmeeinheit sowie das darin aufgenommene Behältnis samt Substrat zu übertragen, wobei die zweite Bewegung eine Walkbewegung ist. Die erste und zweite Bewegungseinrichtung sind auf einer oder mehreren stabilen (positionsfesten) Bodenplatten assembliert.

Die Auftauvorrichtung kann zum parallelen Auftauen einer Mehrzahl von in einzelnen Behältnissen enthaltenen Substraten, insbesondere Blutplasma, eingerichtet sein. Die nachfolgenden Ausführungen beziehen sich zur Vereinfachung auf die Beschreibung jener Konstruktionsmerkmale, die zum Auftauen von in einem einzelnen Beutel enthaltenem Substrat benötigt werden. In der Auftauvorrichtung können die beschriebenen Merkmale also ohne weiteres in einer Mehrzahl vorhanden sein, d.h. die Auftauvorrichtung kann mehrere Auftaueinheiten umfassen, in welchen jeweils ein in einem einzelnen Behältnis enthaltenes Substrat auftaubar ist. Die nachfolgenden Ausführungen beziehen sich also auf eine einzelne Auftaueinheit.

Unter einem "Substrat" kann im Sinne der Erfindung insbesondere ein medizinisches Substrat zu verstehen sein. Ein "medizinisches" Substrat wird vornehmlich im Bereich der Medizin verwendet, z.B. zur intravenösen oder Injektionsbehandlung von Patienten. Um das medizinische Substrat verwenden zu können (z.B. zur Verabreichung an einen Patienten) muss dieses in einer flüssigen Form vorliegen. Dabei muss es sich nicht um eine sich aus einer einzigen chemischen Verbindung zusammensetzende (reine) Flüssigkeit im eigentlichen (chemischen) Sinne handeln. Es kann sich bei dem Substrat beispielsweise um ein homogenes oder heterogenes Stoffgemisch handeln. Als Beispiel für ein homogenes Stoffgemisch ist die Lösung zu nennen. Heterogene Stoffgemische können Emulsionen, Suspensionen oder Schäume sein. Auch können feine Partikel (z.B. Nanopartikel, Proteine etc.) als Feststoffe in einer Flüssigkeit oder einem Gemisch mehrerer Flüssigkeiten verteilt sein. Unter einer "Flüssigkeit" kann hierbei auch eine pastöse oder zähe Masse verstanden sein, die beispielweise eine im Vergleich zu Wasser deutlich herabgesetzte Fließfähigkeit aufweist. Wie erwähnt, befindet sich das Substrat vor dem Auftauvorgang in einem gefrorenen Aggregatszustand. Um einem Auftauvorgang in erfindungsgemäßen Auftauvorrichtung zu erfahren, kann das Substrat vollständig oder partiell gefroren sein. Es ist beispielsweise vorstellbar, dass das Substrat vor dem Auftauen in der Auftauvorrichtung einem Vorab-Auftauprozess unterzogen wird, beispielsweise durch Auftauen bei Raumtemperatur, sodass das Substrat vor dem eigentlichen Auftauen in der Auftauvorrichtung bereits zu einem bestimme Maß aufgetaut ist. Insbesondere kann unter einem "Substrat" ein Blutpräparat wie Blutplasma verstanden werden.

Gleichwohl kann es sich bei einem "Substrat" aber auch um ein anderweitiges Substrat handeln, z.B. ein Nahrungsmittel, ein Futtermittel, eine Chemikalie, ein medizinischer Analyt oder dergleichen.

Das Substrat ist in einem Behältnis enthalten bzw. gelagert. Das Behältnis kann luftdicht verschlossen oder gar unter Vakuumverschluss gehalten sein. Ferner kann sich in dem Behältnis zusätzlich zu dem Substrat ein Inertgas (z.B. N₂) befinden. Das Behältnis kann eine Öffnungsvorrichtung aufweisen, welche einmalig oder mehrfach geöffnet und wieder verschlossen werden kann. Bei dem Behältnis kann es sich insbesondere um einen Beutel, vorzugsweise um einen medizinischen Beutel handeln. Häufig handelt es sich dabei um medizinische Einmalbeutel aus Kunststoff.

Die besonderen Vorteile einer erfindungsgemäßen Auftauvorrichtung seien nachfolgend am Beispiel des Auftauvorgangs von Blutplasma als aufzutauendem Substrat erläutert. Bei den aus dem Stand der Technik bekannten Auftauvorrichtungen (ohne Mikrowellenerwärmung), also z.B. bei der Vorrichtung gemäß DE 195 48 826 A1, wird die oberste Schicht des gefrorenen Blutplasmas beim Auftauen zunächst relativ schnell erwärmt. Dabei bildet sich ein dünner Flüssigkeitsfilm an der Oberfläche des Blutplasmas, welches in diesem verflüssigten Zustand verbleibt. Dies hat zur Folge, dass sich die ursprüngliche Temperaturdifferenz zwischen Flüssigkeitsfilm und Heizelement drastisch verringert. Entsprechend kann ausgehend vom Heizelement die Wärme in Richtung des Inneren (noch gefrorenen) Blutplasmas nur unzureichend nachgeführt werden, um auch die inneren Schichten des Blutplasmas ausreichend mit Wärme zu versorgen und zügig zum Auftauen zu bringen. Dies resultiert daraus, dass mittels in der Auftauvorrichtung vorhandener und einem Heizregelsystem zugehörigen Temperatursensoren im Wesentlichen nur die Temperatur des äußeren (bereits geschmolzenen) Flüssigkeitsfilms erfasst wird. Da die Wärmezufuhr jedoch auf Basis der gemessenen Temperaturdifferenz zwischen geschmolzenem Blutplasma (gemessen durch die Temperatursensoren) und dem Heizelement geregelt wird, bleibt die Temperaturdifferenz zwischen dem Inneren des Blutplasmas und dem Heizelement unberücksichtigt. Folglich wird dem System zu wenig Wärme zugeführt, um ein hinreichend schnelles Auftauen des Blutplasmas zu ermöglichen.

Beim Auftauen von Blutplasma (oder anderer Substrate) mit einer erfindungsgemäßen Auftauvorrichtung hingegen wird durch die auf das Blutplasma ausgeübten Bewegungen (die über die erste und zweite Bewegungseinrichtung ausgeübte erste Bewegung respektive zweite Bewegung) gewährleistet, dass bereits verflüssigtes (aufgetautes) Blutplasma kontinuierlich abkühlt und sich auf Temperaturen von 0°C bis 5 °C einpendelt. Die Bewegungen werden solange ausgeführt, bis das gesamte Blutplasma verflüssigt (aufgetaut) ist. Der besondere Vorteil dieser Vorgehensweise ist die dabei entstehende (weitgehend größere) Temperaturdifferenz zwischen Blutplasma und den Heizelement(en) der Auftauvorrichtung. Somit kann der Wärmenachtransport in das Innere des Substrats (z.B. des Blutplasmas) effizienter nachgeliefert und bezogen auf das Gesamtvolumen des in dem Behältnis enthaltenen Substrats gleichmäßiger verteilt werden. Dadurch wird die Auftauzeit im Vergleich zu den aus dem Stand der Technik bekannten Auftauvorrichtungen weiter verringert. Bezogen auf ein Volumen von 300 mL Blutplasma als Substrat, enthalten in einem Plasmabeutel als Behältnis, können mit einer erfindungsgemäßen Auftauvorrichtung Auftauzeiten von 5 - 8 Minuten erreicht werden, insbesondere jedoch Auftauzeiten von 6 - 8 Minuten.

Die erwähnte Aufnahmeeinheit ist zur temporären Anordnung und/oder Befestigung des Behältnisses (samt darin enthaltenem Substrat) ausgebildet. Die Aufnahmeeinheit kann das Behältnis in einem anwendungsgemäßen Zustand teilweise oder vollständig umschließen. Die Aufnahmeeinheit kann ein- oder mehrteilig ausgebildet sein. Wie erwähnt, umfasst die Aufnahmeeinheit eine Heizeinrichtung zur Bereitstellung von zum Auftauen des Substrats benötigter Wärmeenergie. Insbesondere handelt es sich bei der Heizeinrichtung um eine Einrichtung zur Abgabe von Wärmestrahlung, vorzugsweise jedoch nicht um Mikrowellenstrahlung. Vorteilhaft kann es sein, wenn die Heizeinrichtung derart ausgebildet oder angeordnet ist, dass es ein in der Aufnahmeeinheit platziertes Behältnis gleichmäßig umgibt. Eine gleichmäßige Wärmübertragung ist vorteilhaft zur Gewährleistung eines schonenden und zügigen Auftauvorgangs. Die Heizeinrichtung ist an eine Steuer- und Regelungseinrichtung angebunden. Ferner ist der Steuer- und Regelungseinrichtung zumindest ein Temperatursensor, vorzugsweise mehrere Temperatursensoren zugeordnet, vermöge dessen die Temperatur des aufzutauenden Substrats (zumindest im Oberflächenbereich) erfasst werden kann. In Abhängigkeit der gemessenen Temperatur kann die Wärmezufuhr eingestellt werden. Die Steuerung- und Regelung kann automatisiert erfolgen.

Unterhalb der Aufnahmeeinheit kann eine Auffangschale vorgesehen sein, in welcher Feuchtigkeit, überlaufendes Substrat oder dergleichen aufgefangen werden kann. Damit wird vermieden, dass sich Verunreinigungen oder Feuchtigkeit in der Auftauvorrichtung ansammeln. Die Auffangschale kann zum Entleeren aus der Auftauvorrichtung entnommen werden.

Bei der von der ersten Bewegungseinrichtung auf die Aufnahmeeinheit, sowie das darin aufgenommene Behältnis samt Substrat übertragenen ersten Bewegung handelt es sich um eine Schüttelbewegung im Sinne eines in einer Ebene ausgeführten Hin- und Herbewegens. Die Schüttelbewegung wird mit einer Frequenz von ca. 20 Hz ausgeführt und umfasst vorzugsweise eine Bewegung von ca. 10 mm. Die Schüttelbewegung kann eine Linearbewegung sein, jedoch auch eine im Kreis geführte Bewegung. Auch komplexere in einer Ebene ausgeführte Bewegungsabläufe (Bewegungsmuster) sind denkbar. Die Ebene ist eine horizontale Ebene.

Die Bewegung erfolgt wie erwähnt "im Wesentlichen" in einer Ebene. Dies meint, dass die Hauptbewegungsrichtung innerhalb einer Ebene verläuft, gleichwohl aber geringfügige Abweichungen eröffnet sind. Derartige Abweichungen können beispielsweise aus Unwuchten, Gewichtsverteilungen oder dergleichen resultieren.

Bei der von der zweiten Bewegungseinrichtung auf die Aufnahmeeinheit sowie das darin aufgenommene Behältnis samt Substrat ausgeübten zweiten Bewegung handelt es sich - wie erwähnt - um eine Walkbewegung. Beim Ausüben einer "Walkbewegung" (auch "Walken" genannt) wird das Behältnis samt darin enthaltenem Substrat zumindest partiell verformt. Der Vorgang des "Walkens" kann auch als Kneten oder Drücken verstanden werden. Erfindungsgemäß wird sowohl die erste Bewegung als auch die zweite Bewegung (Walkbewegung) parallel ausgeübt und auf die Aufnahmeeinheit sowie das darin aufgenommene Behältnis samt Substrat übertragen. Dadurch wird eine bessere Durchmischung des Substrats beim Auftauen samt einer damit einhergehenden gleichmäßigeren Wärmeübertragung auf das Substrat ermöglicht, einem entscheidenden Faktor zur Reduktion der Auftauzeit.

Weitere vorteilhafte Ausgestaltungen einer erfindungsgemäßen Auftauvorrichtung ergeben sich aus den in den Unteransprüchen angegebenen, sowie den nachfolgend beschriebenen Merkmalen. Auch die in den Unteransprüchen angegebenen Merkmale seien nachfolgend beschrieben. Betont sei an dieser Stelle, dass die nachfolgend beschriebenen Merkmale ohne Weiteres auch vorteilhafte Ausgestaltungsmerkmale des erfindungsgemäßen Verfahrens sein können. Um Wiederholungen zu vermeiden, seien die in Rede stehenden Merkmale nachfolgend nur in Bezug zu der erfindungsgemäßen Auftauvorrichtung beschrieben.

Nach einer ersten vorteilhaften Ausgestaltung der Erfindung kann bei einer erfindungsgemäßen Auftauvorrichtung vorgesehen sein, dass die Aufnahmeeinheit in Form einer Schale ausgebildet ist, wobei die Schale eine erste Halbschale und eine zweite Halbschale umfasst, wobei die erste Halbschale eine untere Halbschale und die zweite Halbschale eine obere Halbschale ist, und wobei die erste und zweite Halbschale lösbar miteinander verbindbar sind. Eine Schalenform ist vorteilhaft mit Blick auf eine sichere Anordnung des Behältnisses in der Aufnahmeeinheit, ohne die Gefahr, dass das Behältnis während der Bewegungsübertragung aus der Aufnahmeeinheit herausbewegt wird. Die Formgebung der Aufnahmeeinheit ist nicht auf eine Schalenform beschränkt. Die Halbschalen können zumindest partiell eine unterschiedliche Biegsamkeit und/oder Verformbarkeit aufweisen. Unter einer "Verformbarkeit" ist insbesondere eine reversible (elastische) Verformbarkeit zu verstehen. So ist es vorteilhaft, wenn die untere Halbschale zumindest partiell eine höhere Biegsamkeit und/oder Verformbarkeit aufweist als die obere Halbschale. Denn die auf die Aufnahmeeinheit, also die Schale, ausgeübte Walkbewegung wird vornehmlich auf die untere Halbschale ausgeübt. Diese Verformbarkeit (Materialflexibilität) ist erforderlich, damit während des Walkens eine flexible Formveränderung des Behältnisses ermöglicht ist. Denn die Form des Behältnisses verändert sich während des Auftauvorgangs permanent.

Nach einer weiteren vorteilhaften Ausgestaltung der Erfindung kann bei einer erfindungsgemäßen Auftauvorrichtung vorgesehen sein, dass die erste und zweite Halbschale an die Form des Behältnisses angepasst sind, wobei die erste und zweite Halbschale das Behältnis bei dessen Anordnung in der Schale zumindest teilweise umgeben. Durch die Formanpassung der ersten und zweiten Halbschale an die Form des Behältnisses wird ein sicherer Sitz des Behältnisses in der Aufnahmeeinheit gewährleistet. Die Form, Größe und der Materialaufbau der ersten und zweiten Halbschale können dahingehend gestaltet sein, dass eine Ausdehnung des Behältnisses beim Auftauen des Substrats kompensierbar ist. Vorzugsweise dehnt sich die erste und zweite Halbschale bei einer etwaigen Volumenexpansion des Substrats um ein zur Volumenexpansion korrespondierendes Maß aus. Alternativ kann vorgesehen sein, dass das Aufnahmevolumen der Aufnahmeeinheit geringfügig größer ist als das Volumen des Behältnisses, sodass das Behältnis in der Aufnahmeeinheit mit einem gewissen Spiel platziert werden kann. Die Halbschalen können beispielsweise eine rechteckförmige Halbschalen-Basis aufweisen, wobei die Schalenform durch an Seitenbereichen der Halbschalen-Basis angrenzende und sich entlang einer Halbschalen-Längsachse erstreckende Seitenwandabschnitte bereitgestellt wird. Vorzugsweise sind jene Seitenwandabschnitte der unteren Halbschale biegsam ausgebildet, während die Seitenwandabschnitte der oberen Halbschale formstabil, also starr, ausgebildet ist.

Nach einer weiteren vorteilhaften Ausgestaltung der Erfindung kann bei einer erfindungsgemäßen Auftauvorrichtung vorgesehen sein, dass an der ersten und zweiten Halbschalte Fixiermittel vorgesehen sind, mit denen das Behältnis fixierbar ist. Die Fixiermittel können beispielsweise in Form von an den jeweiligen Halbschalen ausgebildeten oder angeordneten Laschen oder Haltespangen verwirklicht sein, die mit dazu korrespondierenden, an der jeweils gegenüberliegenden Halbschale angeordneten oder ausgebildeten Fixiermitteln zusammenwirken. Die mit den Laschen oder Haltespangen zusammenwirkenden Fixiermittel können Befestigungsöffnungen, Klettverschlusselemente, Klemmelemente oder dergleichen sein. Auch kann die Fixierung durch Druckknöpfe verwirklicht werden.

Nach einer weiteren vorteilhaften Ausgestaltung der Erfindung kann bei einer erfindungsgemäßen Auftauvorrichtung vorgesehen sein, dass die erste und zweite Halbschale jeweils einen mehrlagigen Schichtaufbau aufweist, wobei jeweils in einer Schicht des Schichtaufbaus einer jeweiligen Halbschale ein Heizelement vorgesehen ist. Die Heizelemente der jeweiligen Halbschalen stellen die Heizeinrichtung bereit. Die Heizelemente können signaltechnisch mit der erwähnten Steuer- und Regelungseinrichtung verbunden sein. Auch weisen die Heizelemente eine elektrische Versorgungsleitung auf. Die Heizelemente können sich über mäanderartig oder spiralartig entlang der Halbschale (insbesondere über die Fläche der Halbschalen-Basis) erstrecken, wobei die Heizelemente beispielsweise aus Edelstahl ausgebildet sein können. Vorzugsweise weisen die Heizelemente eine Dicke von 50 µm auf. Mit Heizelementen aus Edelstahl kann die Breite der Heizelemente sowie die maximale Erwärmung im Vergleich zu herkömmlichen Kupferelementen vergrößert werden. Auch kann das jeweilige Heizelement in Form einer Heizfolie ausgebildet sein.

Der mehrlagige Schichtaufbau der Halbschalen kann sich vorzugsweise wie folgt zusammensetzen. Dem in der Aufnahmeeinheit angeordneten Behältnis zugewandt weisen die Halbschalen eine Außenhaut bzw. eine Auflageschicht zur Auflage des Behältnisses auf. Diese kann vorzugsweise aus Edelstahl gefertigt sein und vorzugsweise eine Dicke von 100 µm bis 150 µm aufweisen. Daran kann sich eine vorzugsweise 50 µm dicke elektrische Isolationsschicht anschließen, die beispielsweise aus einer Polyimid-Folie gefertigt sein kann. An die Isolationsschicht kann sich jene das Heizelement umfassende Schicht anschließen, wobei diese Schicht eine Dicke von 50 µm aufweisen kann. Jene das Heizelement umfassende Schicht kann in Form einer Edelstahlfolie ausgebildet sein, gleichsam aber auch aus einem elektrisch isolierenden Material, in welches ein oder mehrere Heizelemente aus Edelstahl integriert sind. Daran kann sich eine Träger- bzw. Isolationsschicht anschließen, die vorzugsweise aus einem glasfaserverstärkten Kunststoff gebildet sein kann und eine Dicke von 0,3 mm aufweisen kann. Daran kann sich ein Montageträger für die Heizschale anschließen, welcher aus Aluminium gefertigt sein kann und eine Dicke von 0,5 bis 1,0 mm aufweisen kann.

Nach einer weiteren vorteilhaften Ausgestaltung der Erfindung kann bei einer erfindungsgemäßen Auftauvorrichtung vorgesehen sein, dass die erste Bewegungseinrichtung einen Linearantrieb umfasst, der über eine mechanische Koppeleinrichtung mit zumindest einer beweglich gelagerten Führungseinheit in mechanischer Wirkverbindung steht, wobei die zumindest eine Führungseinheit zur Übertragung der ersten Bewegung mit der Aufnahmeeinheit mechanisch wirkverbunden ist. Die mechanische Koppeleinrichtung kann jeglicher Art sein, solange diese zur Übertragung der von dem Linearantrieb erzeugten ersten Bewegung auf die Aufnahmeeinheit geeignet ist.

Auch ist vorstellbar, dass es sich um eine elektrische Koppeleinrichtung handelt. Unter einer "mechanischen Wirkverbindung" können jegliche Verbindungen verstanden werden, vermöge dessen Bewegungsübertragungen zwischen zwei Bauteilen ermöglicht bzw. vermittelt werden können. Eine mechanische Wirkverbindung kann durch eine unmittelbare mechanische Verbindung zweier Bauteile bereitgestellt werden, jedoch auch durch eines oder mehrere zwischen zwei miteinander bewegungsübertragend zu verbindenden Bauteilen angeordneten Zwischenbauteilen. Der Linearantrieb erzeugt eine translatorische Bewegung, welche auf die Aufnahmeeinheit übertragen wird. Dabei kann die Bewegung eine Linearbewegung oder eine anderweitige Bewegung mit einem vorgegebenen Verlauf sein. Bei dem Linearantrieb kann es sich um einen Gewindestangenantrieb (Kugelgewindetrieb), einen Rollengewindetrieb, einen Hydraulikantrieb, einen Pneumatikantrieb oder um einen elektromechanischen Linearantrieb (z.B. einen Linearmotor) handeln.

Nach einer weiteren vorteilhaften Ausgestaltung der Erfindung kann bei einer erfindungsgemäßen Auftauvorrichtung vorgesehen sein, dass die mechanische Koppeleinrichtung ein Kreuzfedergelenk ist, welches zwischen dem Linearantrieb und der zumindest einen Führungseinheit angeordnet und sowohl mit dem Linearantrieb als auch mit der zumindest einen Führungseinheit oder einem damit wirkverbundenen Bauteil über ein Verbindungsmittel, beispielsweise ein Edelstahlband, verbunden ist. Anstelle eines Edelstahlbandes kann das Verbindungsmittel auch ein anderweitiges Kraftübertragungsmittel sein, z.B. ein Zugband, eine Kette, eine Feder oder dergleichen. Kreuzfedergelenke zeichnen sich durch ihre Reibungsfreiheit, Schmierungsfreiheit und Wartungsfreiheit aus.

Nach einer weiteren vorteilhaften Ausgestaltung der Erfindung kann bei einer erfindungsgemäßen Auftauvorrichtung vorgesehen sein, dass der Linearantrieb mit einem mechanischen oder elektrischen Federsystem verbunden ist, welches dazu eingerichtet ist, Laufeigenschaften und Wirkungsgrad des Linearantriebs einzustellen. Die vorangehend erwähnte mechanische Kopplung zwischen dem Linearantrieb und der zumindest einen Führungseinheit (unter Einsatz der mechanischen Koppeleinrichtung) stellt einen mechanischen Schwingkreis bereit. Dieser wird unterstützt durch das genannte mechanische oder elektrische Federsystem, wodurch insbesondere Wirkungsgrad und Laufruhe des Schwingkreises verbessert werden können. Ein mechanisches Federsystem kann eine verstellbare Bandfeder umfassen. Ein elektrisches Federsystem kann einen Trafo mit einem beweglichen Joch umfassen, welches vorzugsweise in Kombination mit einem als Reluktanzmotor ausgebildeten Linearantrieb gekoppelt ist.

Nach einer weiteren vorteilhaften Ausgestaltung der Erfindung kann bei einer erfindungsgemäßen Auftauvorrichtung vorgesehen sein, dass der Linearantrieb ein elektromechanischer Linearantrieb ist, welcher zumindest eine Stator- und eine Läuferanordnung aufweist, wobei die Läuferanordnung über das Verbindungsmittel, insbesondere das Edelstahlband, mit der mechanischen Koppeleinrichtung verbunden ist. Bei dem elektromechanischen Linearantrieb handelt es sich vorzugsweise um einen Linearmotor, mit dem sich translatorische Vorschubbewegungen erzeugen lassen. Bei einem Linearmotor handelt es sich um die lineare Ausführungsform einer rotierenden Maschine, gedanklich vorstellbar als Abwicklung eines bis zur Mitte aufgeschnittenen Rotationsmotors. Er setzt sich zusammen aus einem stromdurchflossenen Primärteil (vergleichbar mit dem Stator eines Rotationsmotors, vorliegend als Statoranordnung bezeichnet), und einem Reaktions- bzw. Sekundärteil (vergleichbar mit dem Rotor eines Rotationsmotors, hier als Läuferanordnung bezeichnet). Linearmotoren können als Synchron-Linearmotoren, Asynchron-Linearmotoren, Schritt-Linearmotoren oder Gleichstrom-Linearmotoren ausgeführt sein. Während die Statoranordnung bei der asynchronen Bauweise mit Kurzschlussstäben bestückt ist, besteht dieses beim Synchronmotor aus Permanentmagneten. Auch kann es sich bei dem Linearantrieb um einen Reluktanzmotor handeln, welcher eine Statoranordnung und eine Läuferanordnung umfasst.

Nach einer weiteren vorteilhaften Ausgestaltung der Erfindung kann bei einer erfindungsgemäßen Auftauvorrichtung vorgesehen sein, dass die zumindest eine Führungseinheit und die Läuferanordnung jeweils auf Führungsstangen beweglich gelagert sind, wobei die Führungsstangen vorzugsweise in einer gemeinsamen Ebene angeordnet sind. Vorzugsweise handelt es sich bei der Lagerung der zumindest einen Führungseinheit und der Läuferanordnung auf den jeweiligen Führungsstangen um Gleitlager. Die Bewegung der Läuferanordnung und der zumindest einen Führungseinheit sind vorzugsweise gegenläufig. Ferner muss gewährleistet sein, dass die Läuferanordnung und die Führungseinheit (samt den daran angebundenen Komponenten) in etwa die gleiche Gewichtsmasse aufweisen, da ansonsten ungewünschte Unwuchten im Bewegungsablauf entstehen können. Für die genannten gegenläufigen Bewegungen wird ein Drehpunkt benötigt, hier bereitgestellt durch die Koppeleinrichtung, im Speziellen das Kreuzfedergelenk. Das Kreuzfedergelenk ist über ein Verbindungsmittel, beispielsweise ein Edelstahlband, kraftschlüssig mit der Läuferanordnung und mit der zumindest einen Führungseinheit oder einem damit wirkverbundenen Bauteil verbunden. Die Führungsstangen können in einer bevorzugten Ausgestaltung horizontal verlaufen und fluchtend zueinander angeordnet sein. Anstelle von die Läuferanordnung und die zumindest eine Führungseinheit führenden, separaten Führungsstangen kann auch eine gemeinsame Führungsstange zur Führung der Läuferanordnung und der zumindest einen Führungseinheit vorgesehen sein.

Nach einer weiteren vorteilhaften Ausgestaltung der Erfindung kann bei einer erfindungsgemäßen Auftauvorrichtung vorgesehen sein, dass die zweite Bewegungseinrichtung zumindest eine zweite Bewegungseinheit umfasst, die dazu eingerichtet ist, eine Hubbewegung auf die Aufnahmeeinheit, insbesondere die untere Halbschale, auszuüben, wobei bei Ausübung der Hubbewegung eine partielle Deformation der Aufnahmeeinheit, insbesondere der unteren Halbschale, in einem Maß bewirkt, das zu einem aufgetauten Anteil des Substrats korrespondiert. Denn nur in einem zumindest teilweise aufgetauten Zustand lässt sich das Substrat deformieren. Je größer der Anteil von aufgetautem Substrat, desto größer kann die auf das Substrat übertragbare Hubbewegung sein. Beim Ausüben der Hubbewegung auf die untere Halbschale wird diese soweit angehoben, wie sich bereits Substrat im Wege des Auftauvorgangs verflüssigt hat. Physikalisch gesehen befindet sich gefrorenes Blutplasma (als Beispiel für ein mit der erfindungsgemäßen Auftauvorrichtung auftaubares Substrat) in einem kristallinen Zustand. Da sich das Blutplasma während des Auftauvorgangs zunächst an der Oberfläche zunehmend erwärmt, geht das Blutplasma in Oberflächennähe zunehmend in einen amorphen Zustand über. Dadurch nimmt die Verformbarkeit des Blutplasmas zu. Die zweite Bewegungseinrichtung kann eine Mehrzahl von zweiten Bewegungseinheiten umfassen, die an unterschiedlichen räumlichen Positionen eine Hubbewegung auf die Aufnahmeeinheit, insbesondere die untere Halbschale ausüben, sodass durch die Mehrzahl von Hubbewegungen eine Walkbewegung erzeugt wird. Die mit den zweiten Bewegungseinheiten an den jeweiligen unterschiedlichen Positionen auf die untere Halbschale ausgeübten Hubbewegungen können dabei an allen Positionen gleichzeitig oder nacheinander ausgeführt werden. Auch kann vorgesehen sein, an einer vorbestimmten Anzahl von Positionen zeitgleich eine Hubbewegung auszuführen. Vorzugsweise werden die Hubbewegungen derart ausgeführt, dass kontinuierlich wechselnde Positionen der unteren Halbschale (und somit des Behältnisses) mit Druck beaufschlagt werden. Die zweiten Bewegungseinheiten können durch eine Steuerelektronik (beispielsweise einem Mikrocontroller) in einem vorgegebenen Schema angesteuert werden, sodass die zweiten Bewegungseinheiten in einer gewünschten Abfolge Hubbewegungen in Richtung der Aufnahmeeinheit sowie dem darin angeordneten Behältnis samt Substrat ausüben können. Durch die Hubbewegungen wird an kontinuierlich wechselnden Positionen Druck auf das Blutplasma ausgeübt, wodurch bewirkt wird, dass sich der amorphe Anteil des Blutplasmas schneller verflüssigt bzw. weiter auftaut als ohne ausgeübte Walkbewegung.

Nach einer weiteren vorteilhaften Ausgestaltung der Erfindung kann bei einer erfindungsgemäßen Auftauvorrichtung vorgesehen sein, dass zwischen der Aufnahmeeinheit, insbesondere der unteren Halbschale, und der zumindest einen zweiten Bewegungseinheit ein Kraftübertragungselement angeordnet ist, welches zur Übertragung der Hubbewegung auf die Aufnahmeeinheit, insbesondere die untere Halbschale, sowohl mit der Aufnahmeeinheit, insbesondere der unteren Halbschale, als auch mit der zumindest einen zweiten Bewegungseinheit verbunden ist. Sind mehrere der zweiten Bewegungseinheiten vorgesehen, so ist es vorteilhaft eine der Anzahl der zweiten Bewegungseinheiten entsprechende Anzahl an Kraftübertragungselementen vorzusehen, und diese jeweils zwischen der Aufnahmeeinheit und den jeweiligen zweiten Bewegungseinheiten anzuordnen und mit beiden Bauteilen zu verbinden. Das Kraftübertragungselement kann mit einer der jeweiligen zweiten Bewegungseinheit zugehörigen Bauteilkomponente, beispielsweise einem Gehäuse, verbunden sein.

In einer alternativen Ausgestaltung kann das Kraftübertragungselement nebst der Hubbewegung auch zur Übertragung der ersten Bewegung vorgesehen sein. Denn die erste Bewegung (Schüttelbewegung) wird über die mechanische Kopplung/Verbindung zwischen Führungseinheit und Aufnahmeeinheit an das in der Aufnahmeeinheit angeordnete Behältnis (samt Substrat) übertragen. Wird die Führungseinheit an das Kraftübertragungselement angebunden, so kann neben auch die erste Bewegung (Schüttelbewegung) über das Kraftübertragungselement an die Aufnahmeeinheit übertragen werden. In diesem Fall kann das Kraftübertragungselement also zur Übertragung der ersten und zweiten Bewegung vorgesehen sein. Auch können in einer weiteren alternativen Ausgestaltung mehrere Führungseinheiten vorgesehen werden, die entweder auf einer gemeinsamen Führungsstange oder separaten (insbesondere parallel angeordneten) Führungsstangen beweglich gelagert sind. Zur synchronen Bewegungsübertragung ausgehend von dem Linearantrieb können die Führungseinheiten mechanisch miteinander verbunden sein. Auch kann vorgesehen sein, dass der Linearantrieb über eine Mehrzahl von mechanischen Koppeleinrichtungen mit den jeweiligen beweglich gelagerten Führungseinheiten in mechanischer Wirkverbindung steht. Bei den mechanischen Koppeleinrichtungen kann es sich jeweils um Kreuzfedergelenke handeln, die zwischen Linearantrieb und den Führungseinheiten angeordnet sein können, und jeweils sowohl mit dem Linearantrieb als auch mit einer jeweiligen Führungseinheit oder einem damit wirkverbundenen Bauteil über ein Verbindungsmittel, z.B. ein Edelstahlband, verbunden sein können. Bei Vorsehen einer Mehrzahl von Führungseinheiten kann eine jede Führungseinheit an ein Kraftübertragungselement angebunden sein.

Nach einer weiteren vorteilhaften Ausgestaltung der Erfindung kann bei einer erfindungsgemäßen Auftauvorrichtung vorgesehen sein, dass das Kraftübertragungselement eine Kugellagerung umfasst, und dass das Kraftübertragungselement über eine Schweißverbindung an der Aufnahmeeinheit, insbesondere der unteren Halbschale befestigt ist. Eine Kugellagerung, insbesondere über ein Kugelgelenk, ist vorteilhaft, da sich die trigonometrischen Gegebenheiten (durch das sich beim Auftauen stets formverändernde Substrat) mit der Kraftübertragung stets verändern. Über eine Kugellagerung können die Kraftübertragungswege an die veränderte Formgebung des in dem Behältnis enthaltenen Substrats angepasst werden. Anstelle einer Schweißverbindung kann auch eine anderweitige Befestigung in Betracht gezogen werden, beispielsweise eine temperaturstabile Klebeverbindung, oder eine mechanische Verbindung (z.B. eine Nietverbindung).

Nach einer weiteren vorteilhaften Ausgestaltung der Erfindung kann bei einer erfindungsgemäßen Auftauvorrichtung vorgesehen sein, dass die zumindest eine zweite Bewegungseinheit zumindest einen Hubmagneten umfasst, welcher dazu eingerichtet ist, durch Ausführen von Hubbewegungen einen Hub, insbesondere einen Hebe- und Senkhub, auf das Kraftübertragungselement, die mit dem Kraftübertragungselement verbundene Aufnahmeeinheit und das in der Aufnahmeeinheit angeordnete Behältnis samt darin enthaltenem Substrat zu übertragen. Umfasst die zweite Bewegungseinrichtung eine Mehrzahl von zweiten Bewegungseinheiten, so kann jede der zweiten Bewegungseinheiten einen Hubmagneten umfassen. Beim Ausführen der Hubbewegung kann der Hub vom Hubmagneten zunächst auf ein der jeweiliges der zweiten Bewegungseinheit zugehöriges Gestänge übertragen werden. Das Gestänge kann mit einem beweglich gelagerten Schlitten verbunden sein, wobei der Schlitten mit dem Kraftübertragungselement verbunden ist. Der Schlitten ist derart beweglich gelagert, dass er ebenfalls die von dem Linearantrieb ausgelöste erste Bewegung (Schüttelbewegung) nachvollzieht. Die bewegliche Lagerung des Schlittens bewirkt, dass die zweiten Bewegungseinheiten (zur Erzeugung der Walkbewegung) von der ersten Bewegung bzw. den zugehörigen Schwingungen entkoppelt sind. Die zweiten Bewegungseinheiten vollziehen die erste Bewegung also vorzugsweise nicht nach. Der Schlitten an einer in einem einer jeweiligen zweiten Bewegungseinheit zugehörigen Gehäuse gelagerten Führungsstange beweglich gelagert sein.

Nach einer weiteren vorteilhaften Ausgestaltung der Erfindung kann bei einer erfindungsgemäßen Auftauvorrichtung vorgesehen sein, dass die zumindest eine zweite Bewegungseinheit zumindest eine mechanische Hubeinrichtung umfasst, die dazu eingerichtet, durch Ausführen von Hubbewegungen einen Hub, insbesondere einen Hebe- und Senkhub, auf einen quer, insbesondere senkrecht, zur Hubachse bewegbar angeordneten Kraftübertragungsstift zu übertragen.

Dabei kann der Kraftübertragungsstift quer, insbesondere senkrecht, zur Hubachse zwischen einer ersten und einer zweiten Stellung bewegbar sein.

in seiner zweiten Stellung eine von der zumindest einen mechanischen Hubeinrichtung ausgeführte Hubbewegung auf das Kraftübertragungselement, die mit dem Kraftübertragungselement verbundene Aufnahmeeinheit und das in der Aufnahmeeinheit angeordnete Behältnis samt darin enthaltenem Substrat zu übertragen. Im Besonderen kann der Kraftübertragungsstift dazu eingerichtet sein, in seiner zweiten Stellung eine von der zumindest einen mechanischen Hubeinrichtung ausgeführte Hubbewegung auf ein unmittelbar oder mittelbar mit dem Kraftübertragungselement verbundenes Bauteil zu übertragen. Insbesondere kann vorgesehen sein, dass der Kraftübertragungsstift in seiner zweiten Stellung die Hubbewegung auf ein Gehäuseteil überträgt, in welchem ein mit dem Kraftübertragungselement verbundener Schlitten beweglich gelagert ist. Der Schlitten kann an einer in dem Gehäuseteil angeordneten Führungsstange beweglich gelagert sein. Die bewegliche Lagerung des Schlittens bewirkt, dass bei dieser Ausgestaltungen die zweiten Bewegungseinheiten (zur Erzeugung der Walkbewegung) von der ersten Bewegung entkoppelt sind.

In der ersten Stellung des Kraftübertragungsstiftes erfolgt keine Übertragung der Hubbewegung in Richtung des Kraftübertragungselements. Der Kraftübertragungsstift kann durch eine Steuereinheit angesteuert werden und über eine Bewegungsmechanik quer, insbesondere senkrecht, zur Hubachse hin- und herbewegt werden. Der Kraftübertragungsstift kann in einer Führungseinrichtung, z.B. einer Führungsnut, beweglich geführt sein. Eine solche Variante verlangt im Gegensatz zu einer auf Hubmagneten basierenden Ausgestaltung einen geringeren Leistungsbedarf. Die Hubbewegung kann bei dieser Ausgestaltung durch einen Getriebemotor erzeugt werden, welcher eine Nockenwelle mit ca. 60 U/min antreibt. Die Bewegung wird zunächst auf einen mit der Nockenwelle verbundenen und entlang der Hubachse linearbeweglich angeordneten Stößel übertragen, wobei der Stößel in der zweiten Stellung des Kraftübertragungsstiftes an einem (unteren) Ende des Kraftübertragungsstiftes angreifen und die Hubbewegung auf diesen übertragen kann.

Wie erwähnt, wird die o.g. Aufgabe zudem mit einem Verfahren zum Auftauen eines in gefrorenem Aggregatszustand befindlichen Substrats, insbesondere Blutplasma gelöst. Dabei ist das Substrat in einem Behältnis, insbesondere einem Beutel, enthalten. Das Verfahren wird mit einer erfindungsgemäßen Auftauvorrichtung ausgeführt und umfassend die folgenden Schritte:
a. Anordnen des das Substrat enthaltenden Behältnisses in der Aufnahmeeinheit,
b. Aufheizen des in dem Behältnis enthaltenen Substrats mit der Heizeinrichtung, wobei während des Aufheizens
   i. mit der ersten Bewegungseinrichtung eine erste Bewegung auf die Aufnahmeeinheit sowie das darin aufgenommene Behältnis samt Substrat übertragen wird, wobei die erste Bewegung eine im Wesentlichen in einer Ebene ausgeführte Bewegung, insbesondere eine Linearbewegung, ist, sowie
   ii. mit der zweiten Bewegungseinrichtung eine zweite Bewegung auf die Aufnahmeeinheit sowie das darin aufgenommene Behältnis samt Substrat übertragen wird, wobei die zweite Bewegung eine Walkbewegung ist.

Weitere Merkmale und Vorteile der Erfindung ergeben sich aus der folgenden Beschreibung nicht einschränkend zu verstehender Ausführungsbeispiele der Erfindung, welche im Folgenden unter Bezugnahme auf die Zeichnungen näher erläutert werden. In diesen Zeichnungen zeigen:
- Fig. 1: eine schematische Darstellung einer erfindungsgemäßen Auftauvorrichtung samt ihren wesentlichen Bestandteilen;
- Fig. 2: eine schematische Darstellung der erfindungsgemäßen Auftauvorrichtung im Längsschnitt, wobei die Darstellung insbesondere jene der ersten Bewegungseinrichtung zugehörigen Komponenten zu erkennen gibt;
- Fig. 3: eine perspektivische Ansicht einer der erfindungsgemäßen Auftauvorrichtung zugehörigen Aufnahmeeinheit samt Behältnis;
- Fig. 4: eine den Schichtaufbau wiedergebende Darstellung einer der Aufnahmeeinheit nach Fig. 3 zugehörigen Halbschale;
- Fig. 5: eine schematische Darstellung einer der zweiten Bewegungseinrichtung zugehörigen zweiten Bewegungseinheit in einer ersten Ausführungsform;
- Fig. 6: eine schematische Darstellung einer der zweiten Bewegungseinrichtung zugehörigen zweiten Bewegungseinheit in einer zweiten Ausführungsform;
- Fig. 7: eine Darstellung einer möglichen räumlichen Anordnung einer Mehrzahl zweiter Bewegungseinheiten nach Fig. 5;
- Fig. 8: eine Darstellung einer möglichen räumlichen Anordnung einer Mehrzahl zweiter Bewegungseinheiten nach Fig. 6;
- Fig. 9: eine schematische Darstellung des Aufbaus eines elektromechanischen Linearantriebs samt elektrischem Federsystem;
- Fig. 10: eine schematische Darstellung des Aufbaus eines Reluktanzmotors als Linearantrieb samt elektrischem Federsystem.

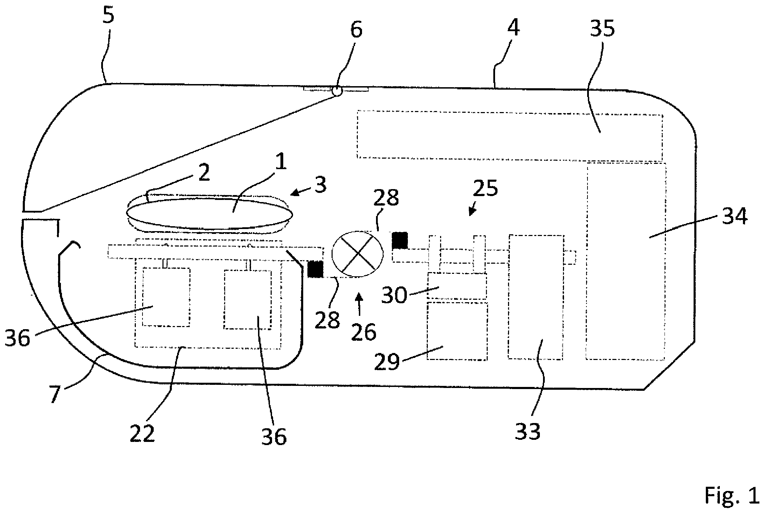

Die Fig. 1 zeigt eine schematische Darstellung des Aufbaus einer erfindungsgemä-ßen Auftauvorrichtung zum Auftauen eines in gefrorenem Aggregatszustand befindlichen Substrats 1. Bei dem Substrat 1 kann es sich insbesondere um Blutplasma handeln. Das gefrorene Substrat 1 ist in einem Behältnis 2, insbesondere einem Beutel, enthalten.

Die Auftauvorrichtung weist ein Gehäuse 4 auf, welches über eine Öffnungsklappe 5 geöffnet werden kann. Die Öffnungsklappe 5 ist über ein Scharnier 6 an dem Gehäuse 4 angelenkt bzw. schwenkbar gelagert. Im Inneren des Gehäuses 5 ist eine Aufnahmeeinheit 3 angeordnet, in welcher das Behältnis 2 (samt Substrat 1) angeordnet bzw. eingelegt werden kann. Für die nachfolgenden Erläuterungen sei davon ausgegangen, dass es sich bei dem Behältnis 2 um einen formflexiblen Blutplasmabeutel handelt. Die Aufnahmeeinheit 3 umfasst eine Heizeinrichtung (in Fig. 1 nicht dargestellt) zur Bereitstellung von zum Auftauen des Substrats benötigter Wärmeenergie. Die Aufnahmeeinheit 3 ist in Form einer Schale ausgebildet, wobei die Schale eine erste Halbschale 8 und eine zweite Halbschale 9 umfasst. Die erste Halbschale 8 stellt dabei eine untere Halbschale und die zweite Halbschale 9 eine obere Halbschale bereit. Die Halbschalen 8, 9 können lösbar miteinander verbunden werden. Die erste Halbschale 8 und zweite Halbschale 9 sind an die Form des Behältnisses 2 angepasst, wobei die erste und zweite Halbschale 8, 9 das Behältnis 2 bei dessen Anordnung in der Schale zumindest teilweise umgeben (vgl. Fig. 1).

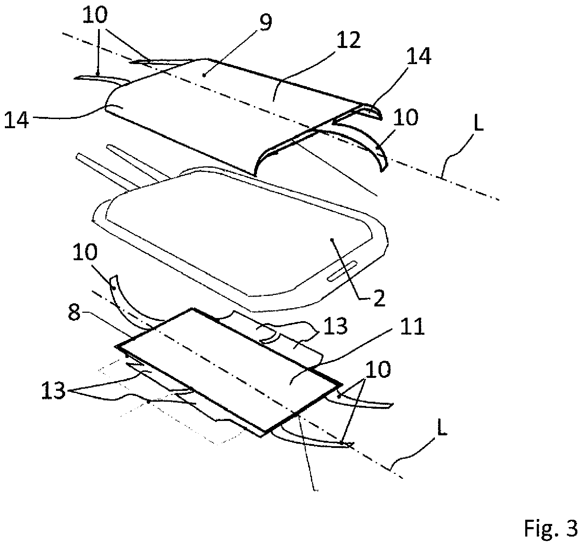

Wie in der Fig. 3 im Detail gezeigt, sind an der ersten und zweiten Halbschale 8, 9 Fixiermittel 10 in Form von Haltespangen angeordnet, mit denen das Behältnis 2 in der von den Halbschalen 8, 9 gebildeten Schale fixierbar ist. Ferner können die Halbschalen 8, 9 mittels der Fixiermittel 10 miteinander verbunden werden. Die Halbschalen 8, 9 weisen eine rechteckförmige Halbschalen-Basis 11, 12 auf, wobei die Schalenform durch an Seitenbereichen der Halbschalen-Basis 11, 12 angrenzende und sich entlang einer Halbschalen-Längsachse L erstreckende Seitenwandabschnitte 13, 14 bereitgestellt wird. Vorzugsweise sind jene Seitenwandabschnitte 13 der unteren Halbschale 8 biegsam ausgebildet, während die Seitenwandabschnitte 14 der oberen Halbschale 9 formstabil, also starr, ausgebildet sind. Die in Form von Haltespangen ausgebildeten Fixiermittel 10 können durch geeignete Befestigungsvorrichtungen an den gegenüberliegenden Halbschalen 8, 9 befestigt werden.

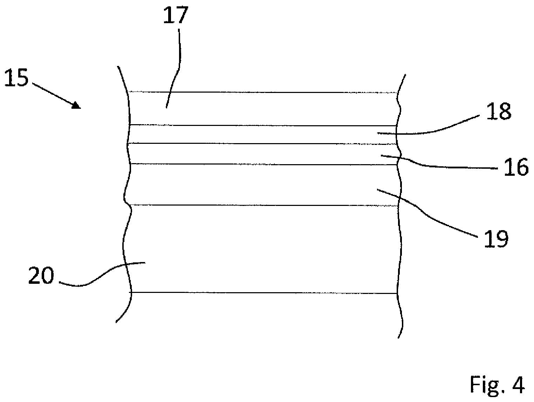

Die erste und zweite Halbschale 8, 9 weisen jeweils einen mehrlagigen Schichtaufbau 15 auf (Fig. 4), wobei jeweils in einer Schicht des Schichtaufbaus 15 einer jeweiligen Halbschale 8, 9 ein Heizelement 16 vorgesehen ist. Die Heizelemente 16 der jeweiligen Halbschalen 8, 9 stellen die Heizeinrichtung bereit. Die Heizelemente 16 können sich mäanderartig oder spiralartig entlang der Halbschale 8, 9 (insbesondere über die Fläche der Halbschalen-Basis 11, 12) erstrecken, wobei die Heizelemente 16 aus Edelstahl ausgebildet sind. Vorzugsweise weisen die Heizelemente 16 eine Dicke von 50 µm auf. Auch kann das jeweilige Heizelement 16 in Form einer Heizfolie ausgebildet sein.

Der mehrlagige Schichtaufbau 15 der Halbschalen 8, 9 setzt sich wie folgt zusammen (vgl. Fig. 4). Dem in der Aufnahmeeinheit 3 angeordneten Behältnis 2 zugewandt weisen die Halbschalen 8, 9 eine Außenhaut 17 bzw. eine Auflageschicht zur Auflage des Behältnisses 2 auf. Diese ist vorzugsweise aus Edelstahl gefertigt und weist eine Schichtdicke von 100 µm bis 150 µm auf. Daran schließt sich eine vorzugsweise 50 µm dicke elektrische Isolationsschicht 18 an, die beispielsweise aus einer Polyimid-Folie gefertigt sein kann. An die Isolationsschicht 18 schließt sich jene das Heizelement 16 umfassende Schicht an, wobei diese Schicht eine Dicke von 50 µm aufweisen kann. Jene das Heizelement 16 umfassende Schicht kann auch in Form einer Edelstahlfolie ausgebildet sein, gleichsam aber auch aus einem elektrisch isolierenden Material, in welches ein oder mehrere Heizelemente 16 aus Edelstahl integriert sind. Daran schließt sich eine Träger- bzw. Isolationsschicht 19 an, die vorzugsweise aus einem glasfaserverstärkten Kunststoff gebildet ist und eine Dicke von 0,3 mm aufweist. Daran schließt sich ein Montageträger 20 für die jeweilige Halbschale 8, 9 an, welcher aus Aluminium gefertigt ist und eine Dicke von 0,5 bis 1,0 mm aufweist.

Wie in der Fig. 1 gezeigt, ist ein weiterer Bestandteil der Auftauvorrichtung die in dem Gehäuse 4 unterhalb der Aufnahmeeinheit 3 angeordnete Auffangschale 7, welche verhindert, dass im Wege des Auftauvorgangs entstehende Feuchtigkeit oder anderweitige Verunreinigungen (beispielsweise durch eine undichte Stelle des Behältnisses 2 entweichendes Substrat 1) an Stellen der Auftauvorrichtung gelangt, an welchen beispielsweise elektrische Komponenten beschädigt werden könnten. Die Auffangschale 7 ist vorzugsweise dahingehend ausgebildet und angeordnet, dass sie zum Entleeren aus dem Gehäuse 4 entnommen werden kann.

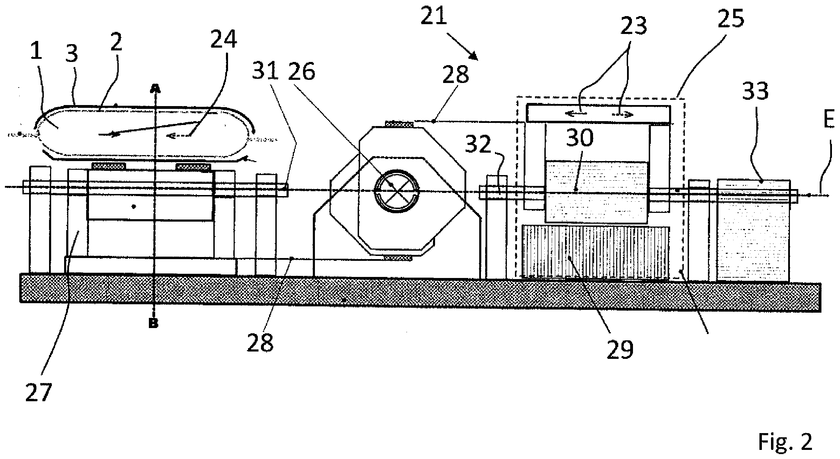

In dem Gehäuse 4 ist eine erste Bewegungseinrichtung 21 angeordnet, die dazu eingerichtet ist, eine erste Bewegung (gekennzeichnet durch die Bewegungspfeile 24) auf die Aufnahmeeinheit 3 sowie das darin aufgenommene Behältnis 2 samt Substrat 1 zu übertragen. Die erste Bewegung ist eine im Wesentlichen in einer Ebene ausgeführte Bewegung, insbesondere eine Linearbewegung. Die erste Bewegungseinrichtung 21 umfasst einen Linearantrieb 25, der über eine mechanische Koppeleinrichtung 26 mit zumindest einer beweglich gelagerten Führungseinheit 27 in mechanischer Wirkverbindung steht (Fig. 2), wobei die zumindest eine Führungseinheit 27 zur Übertragung der ersten Bewegung mit der Aufnahmeeinheit 3 mechanisch wirkverbunden ist.

Die mechanische Koppeleinrichtung 26 ist ein Kreuzfedergelenk, welches zwischen dem Linearantrieb 25 und der zumindest einen Führungseinheit 27 angeordnet und sowohl mit dem Linearantrieb 25 als auch mit der zumindest einen Führungseinheit 27 oder einem damit wirkverbundenen Bauteil über ein Verbindungsmittel 28, beispielsweise ein Edelstahlband, verbunden ist. Der Linearantrieb 25 ist ein elektromechanischer Linearantrieb ist, welcher zumindest eine Statoranordnung 29 und eine Läuferanordnung 30 aufweist, wobei die Läuferanordnung 30 über das Verbindungsmittel 28, insbesondere das Edelstahlband, mit der mechanischen Koppeleinrichtung 26 verbunden ist. Die zumindest eine Führungseinheit 27 und die Läuferanordnung 30 sind jeweils auf Führungsstangen 31, 32 beweglich gelagert, wobei die Führungsstangen 31, 32 in einer gemeinsamen Ebene E angeordnet sind. Wird die Läuferanordnung 30 in Bewegung versetzt (vgl. die Bewegungspfeile 23) so wird diese Bewegung durch die mechanische Koppeleinrichtung 26 an die Führungseinheit 27 übertragen. Die Führungseinheit 27 erfährt ebenfalls eine Bewegung (vgl. die Bewegungspfeile 24), die jedoch gegenläufig zu jener Bewegung der Läuferanordnung 30 ist.

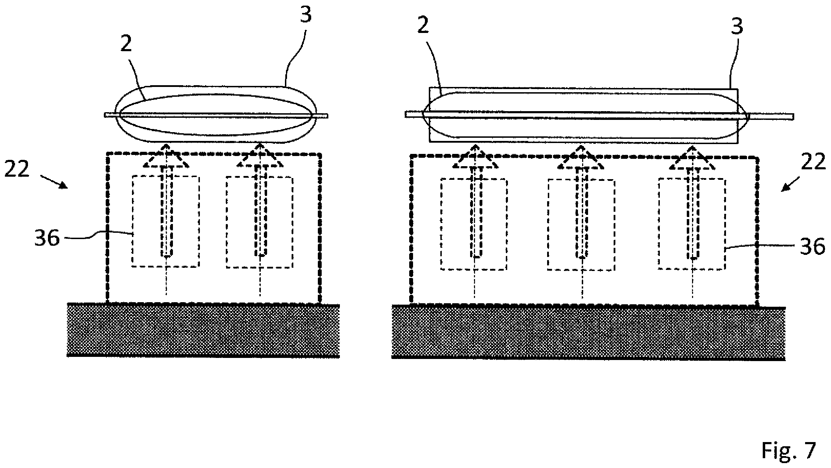

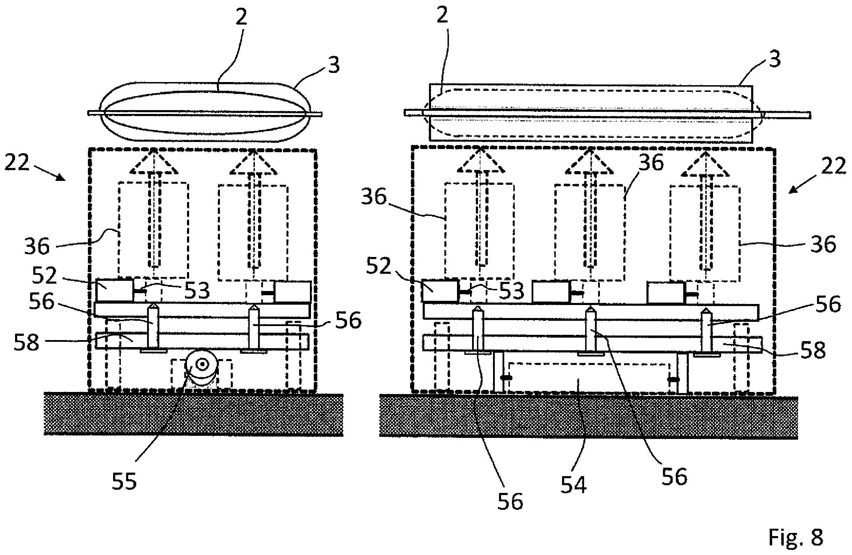

Ferner weist die Auftauvorrichtung eine zweite Bewegungseinrichtung 22 auf, die dazu eingerichtet ist, eine zweite Bewegung auf die Aufnahmeeinheit 3 sowie das darin aufgenommene Behältnis 2 samt Substrat 1 zu übertragen, wobei die zweite Bewegung eine Walkbewegung ist. In der Fig. 1 ist schematisch dargestellt, dass die zweite Bewegungseinrichtung 22 eine Mehrzahl zweiter Bewegungseinheiten 36 aufweist, die an mehreren Positionen eine Bewegung auf die Aufnahmeeinheit 3 übertragen. Gleiches ist in den Figuren 7 und 8 wiedergegeben.

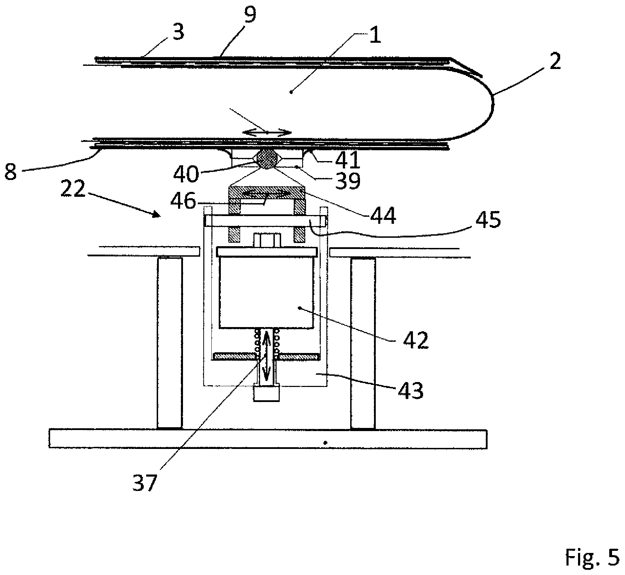

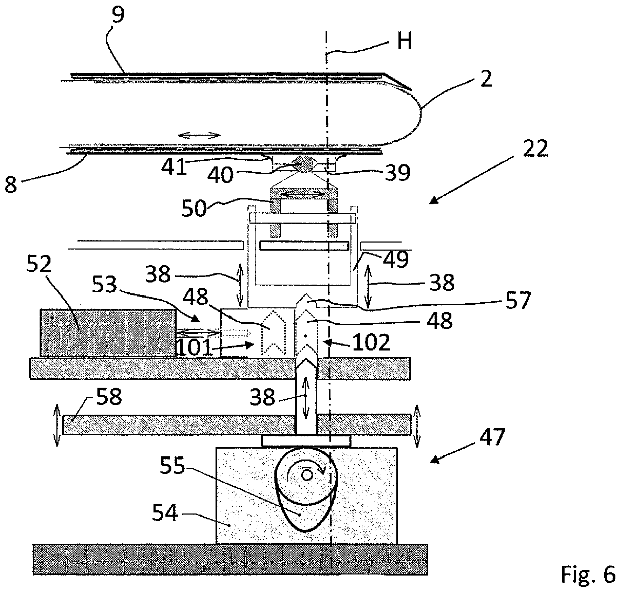

Die zweite Bewegungseinrichtung 22 umfasst also zumindest eine, vorzugsweise mehrere, zweite Bewegungseinheit(en) 36, wobei die zumindest eine zweite Bewegungseinheit 36 dazu eingerichtet ist, eine Hubbewegung (vgl. z.B. den Bewegungspfeil 37 in Fig. 5 und die Bewegungspfeile 38 in Fig 6, allesamt vertikale Hubbewegungen kennzeichnend) auf die Aufnahmeeinheit 3, insbesondere die untere Halbschale 8, auszuüben, wobei bei Ausübung der Hubbewegung eine partielle Deformation der Aufnahmeeinheit 3, insbesondere der unteren Halbschale 8, in einem Maß bewirkt wird, das zu einem aufgetauten Anteil des Substrats 1 korrespondiert.

Die zweiten Bewegungseinheiten 36 sind an unterschiedlichen räumlichen Positionen angeordnet, um eine Hubbewegung auf die Aufnahmeeinheit 3, insbesondere die untere Halbschale 8, auszuüben, sodass durch die Mehrzahl von Hubbewegungen eine Walkbewegung erzeugt wird.

In den Figuren 5 und 6 sind unterschiedliche Ausgestaltungen von zweiten Bewegungseinheiten 36 dargestellt. Beide Varianten ist jedoch gemein, dass zwischen der Aufnahmeeinheit 3, nämlich der unteren Halbschale 8, und der zumindest einen zweiten Bewegungseinheit 36 ein Kraftübertragungselement 39 angeordnet ist, welches zur Übertragung der Hubbewegung auf die Aufnahmeeinheit 3, nämlich die untere Halbschale 8, sowohl mit der Aufnahmeeinheit 3 (der unteren Halbschale 8), als auch mit der zumindest einen zweiten Bewegungseinheit 36 verbunden ist. Das Kraftübertragungselement 39 weist eine Kugellagerung 40 auf und ist über eine Schweißverbindung 41 an der unteren Halbschale 8 befestigt.

Gemäß dem Ausführungsbeispiel nach Fig. 5 umfasst die zumindest eine zweite Bewegungseinheit 36 zumindest einen Hubmagneten 42, der dazu eingerichtet ist, durch Ausführen von Hubbewegungen (vgl. den Bewegungspfeil 37) einen Hub, insbesondere einen Hebe- und Senkhub, auf das Kraftübertragungselement 39, die mit dem Kraftübertragungselement 39 verbundene Aufnahmeeinheit 3 und das in der Aufnahmeeinheit 3 angeordnete Behältnis 2 samt darin enthaltenem Substrat 1 zu übertragen. Umfasst die zweite Bewegungseinrichtung 22 eine Mehrzahl von zweiten Bewegungseinheiten 36, so kann jede der zweiten Bewegungseinheiten 36 einen Hubmagneten umfassen.

Beim Ausführen der Hubbewegung (Fig. 5) wird der Hub vom Hubmagneten 42 zunächst auf ein der zweiten Bewegungseinheit 36 zugehöriges Gestänge 43 (oder ein Gehäuseteil) übertragen. Das Gestänge 43 (oder Gehäuseteil) ist mit einem beweglich gelagerten Schlitten 44 verbunden, wobei der Schlitten 44 wiederum mit dem Kraftübertragungselement 39 verbunden ist. Der Schlitten 44 ist an einer Führungsstange 45 derart beweglich gelagert, dass er die von dem Linearantrieb 25 ausgelöste erste Bewegung (Schüttelbewegung) nachvollzieht (siehe den Bewegungspfeil 46). Die bewegliche Lagerung des Schlittens 44 bewirkt, dass die zweiten Bewegungseinheiten 36 (zur Erzeugung der Walkbewegung) von der ersten Bewegung bzw. den zugehörigen Schwingungen entkoppelt sind. Die zweiten Bewegungseinheiten 36 vollziehen die erste Bewegung also nicht nach.

Gemäß dem Ausführungsbeispiel nach Fig. 6 umfasst die zumindest eine zweite Bewegungseinheit 36 zumindest eine mechanische Hubeinrichtung 47, die dazu eingerichtet, durch Ausführen von Hubbewegungen einen Hub, insbesondere einen Hebe- und Senkhub, auf einen quer, insbesondere senkrecht, zur Hubachse H bewegbar angeordneten Kraftübertragungsstift 48 zu übertragen.

Dabei ist der Kraftübertragungsstift 48 quer, insbesondere senkrecht, zur Hubachse H zwischen einer ersten Stellung 101 und einer zweiten Stellung 102 bewegbar. In seiner zweiten Stellung 102 überträgt der Kraftübertragungsstift 48 eine von der mechanischen Hubeinrichtung 47 erzeugte Hubbewegung auf das Kraftübertragungselement 39, die mit dem Kraftübertragungselement 39 verbundene Aufnahmeeinheit 3 und das in der Aufnahmeeinheit 3 angeordnete Behältnis 2 samt darin enthaltenem Substrat 1. im Besonderen ist der Kraftübertragungsstift 48 dazu eingerichtet, in seiner zweiten Stellung 102 eine von der mechanischen Hubeinrichtung 47 erzeugte Hubbewegung auf ein Gehäuseteil 49 zu übertragen, in welchem ein mit dem Kraftübertragungselement 39 verbundener Schlitten 50 beweglich gelagert ist. Der Schlitten 50 ist an einer in dem Gehäuseteil 49 angeordneten Führungsstange 51 beweglich gelagert. Die bewegliche Lagerung des Schlittens 50 bewirkt, dass die zweite Bewegungseinheit 36 von der ersten Bewegungseinrichtung 21 und den davon erzeugten ersten Bewegungen entkoppelt ist.

In der ersten Stellung 101 des Kraftübertragungsstiftes 48 erfolgt keine Übertragung der Hubbewegung in Richtung des Kraftübertragungselements 39. Der Kraftübertragungsstift 48 wird durch eine Steuereinheit 52 angesteuert und über eine Bewegungsmechanik 53 quer, insbesondere senkrecht, zur Hubachse hin- und herbewegt. Die Hubbewegung wird bei dieser Ausgestaltung durch einen Getriebemotor 54 erzeugt, welcher eine Nockenwelle 55 mit ca. 60 U/min antreibt. Die Bewegung wird zunächst auf einen mit der Nockenwelle 55 verbundenen und entlang der Hubachse H linearbeweglich angeordneten Stößel 56 übertragen, wobei der Stößel 56 in der zweiten Stellung 102 des Kraftübertragungsstiftes 48 an einem (unteren) Ende des Kraftübertragungsstiftes 48 angreift und die Hubbewegung auf diesen übertragen kann. Am unteren Ende des Gehäuseteils 49 ist indes eine Ausnehmung 57 ausgebildet, die zu einem oberen Ende des Kraftübertragungsstiftes 48 korrespondiert und einen Formschluss ermöglicht. Zur Anordnung einer Mehrzahl von Stößeln 56 kann eine Stößelplatte 58 vorgesehen sein.

Die Fig. 7 veranschaulicht wie die zweiten Bewegungseinheiten 36 (gemäß der Ausführungsvariante nach Fig 5 samt Hubmagneten 42) über die Breite der Aufnahmeeinheit 3 (Darstellung links) und über die Länge der Aufnahmeeinheit 3 verteilt bzw. angeordnet werden können. Insgesamt sind bei dieser Variante sechs zweite Bewegungseinheiten 36 mit insgesamt sechs Hubmagneten 42 vorgesehen.

Die Fig. 8 veranschaulicht in Analogie zur Fig. 7 ebenfalls eine Verteilung der zweiten Bewegungseinheiten 36, jedoch bei einer Ausführungsvariante nach Fig. 6 (mechanische Hubeinrichtung 47). In der linken Darstellung ist die Verteilung der zweiten Bewegungseinheiten 36 über die Breite der Aufnahmeeinheit 3 wiedergegeben, während in der rechten Darstellung die Verteilung der zweiten Bewegungseinheiten 36 über die Länge der Aufnahmeeinheit 3 dargestellt ist. Wie dargestellt, sind sechs Stößel 56 auf einer gemeinsamen Stößelplatte 58 angeordnet. Die Stößelplatte 58 und damit die Stößel 56 werden unter Einsatz eines Getriebemotors 54 und einer Nockenwelle 55 in eine Hubbewegung versetzt. Jedem Stößel 56 ist - wie im Ausführungsbeispiel der Fig. 6 beschrieben - ein Kraftübertragungsstift 48 zugeordnet, der jeweils eine erste Stellung 101 und eine zweite Stellung 102 einnehmen kann.

Entsprechend ist jedem der sechs Kraftübertragungsstifte 48 eine Steuereinheit 52 und eine Bewegungsmechanik 53 zugeordnet.

Der Linearantrieb 25 ist mit einem mechanischen oder elektrischen Federsystem 33 verbunden, welches dazu eingerichtet ist, Laufeigenschaften und Wirkungsgrad des Linearantriebs 25 einzustellen. Insbesondere ist das Federsystem 33 dazu vorgesehen, den Schwingkreis des Schüttelantriebs (erste Bewegungseinrichtung) zu verbessern und gleichzeitig für einen ruhigeren Lauf zu sorgen.

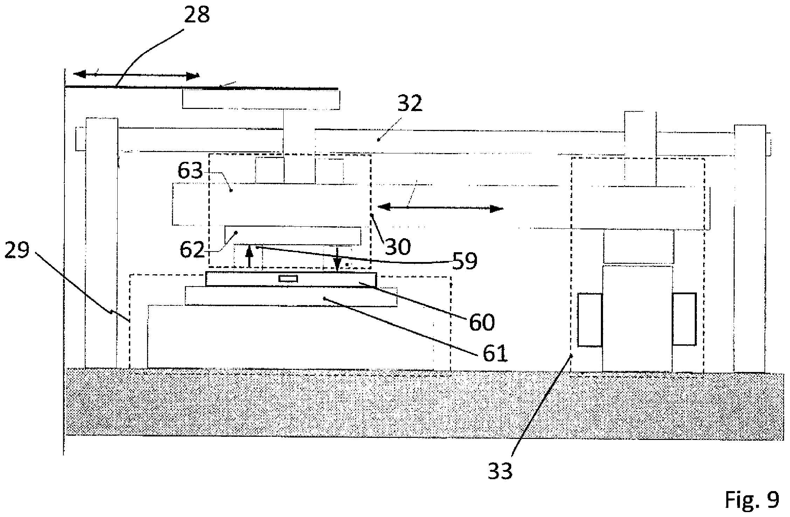

In der Fig. 9 ist ein elektrisches Federsystem in Kombination mit einem elektromechanischen Linearantrieb 25 wiedergegeben, wobei es sich im Speziellen um einen Permanentmagnet-Linearmotor als Linearantrieb 25 handelt. Ein solcher Linearantrieb 25 umfasst die folgenden Komponenten: Neodymium-Permanentmagnete 59, eine Cu-Flachspule 60, einen geblechten Fe-Stator-Rückschluss 61, einen geblechten Fe-Läufer-Rückschluss 62 sowie ein Läuferlager 63 mit Gleitlagern. Das elektrische Federsystem 33 ist über das Läuferlager 63 mit dem Permanentmagnet-Linearmotor verbunden bzw. mit diesem wirkgekoppelt.

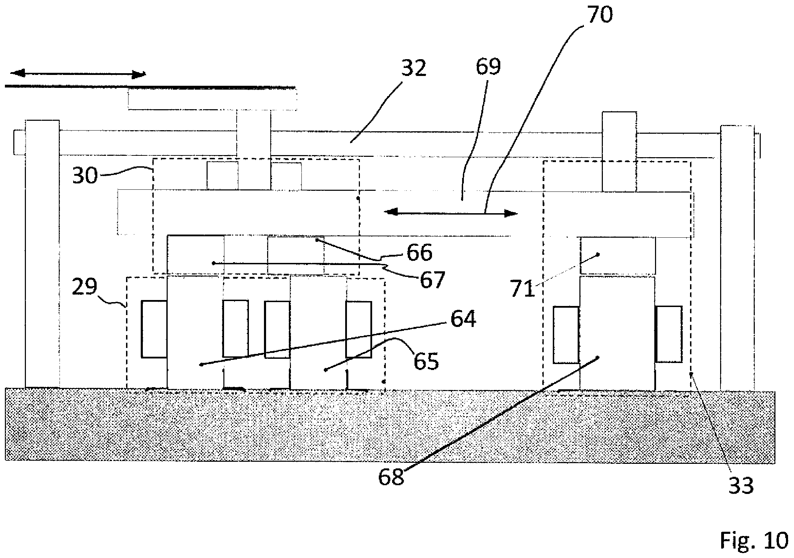

In der Fig. 10 ist das elektrische Federsystem 33 in Verbindung mit einem Reluktanzmotor dargestellt, welcher den Permanentmagnet-Linearmotor nach Fig. 9 ersetzt. Reluktanzmotoren sind aus dem Stand der Technik allgemein bekannt und betreffen eine besondere Bauform eines Elektromotors, bei der ein Kraftmoment allein durch Reluktanzkraft erzeugt wird und nicht zu wesentlichen Anteilen durch die Lorentzkraft, wie es bei magnetisch erregten Maschinen der Fall ist.

In Kombination mit dem elektrischen Federsystem 33 fungiert wie der Reluktanzmotor. Der in Fig. 11 links dargestellte Trafo 64 verhält sich magnetisch neutral und verbleibt ohne Strom. Dem linken Trafo 64 ist ein geblechtes Joch 67 zugeordnet. Der rechte Trafo 65 wird zu einem bestimmten Zeitpunkt mit Strom versorgt, sodass eine Kraft, proportional zur Höhe des applizierten Stroms, nach rechts entsteht und der linke Trafo 64 dabei gleichzeitig ohne Stromversorgung verbleibt. Hat nun das Joch 66 des rechten Trafos 65 eine neutrale Position erreicht, so wiederholt sich der vorangehend beschriebene Ablauf, jedoch in umgekehrter Richtung. Das elektrische Federsystem 33 (Trafo 67) weist eine etwas größere Dimensionierung auf als die Reluktanz-Trafos 64, 65. Der Trafo 68 des Federsystems 33 wird kontinuierlich unter Strom gesetzt, was zur Folge hat, dass dieser (trotz Stromfluss) in neutraler Position und keine Kräfte ausübt (vgl. die Jochposition des geblechten Jochs 71). Bewegt nun der Reluktanzmotor den Schlitten 69 (vgl. den Bewegungspfeil 70), so erzeugt das Federsystem 33 eine Kraft, die der Kraft des Reluktanzmotors entgegenwirkt. Entscheidend dabei ist, dass der Strom des Federsystems so eingestellt wird, dass das gesamte Antriebssystem durch die Federkraft unterstützt wird und bei geringstem Stromfluss des Reluktanzmotors die maximale mechanische Auslenkung erzielt wird.

Weitere Bestandteile der Auftauvorrichtung sind - wie in Fig. 1 gezeigt - Einrichtungen der Leistungselektronik 34 und Elektronik 35.

### Bezugszeichenliste

- 1: Substrat
- 2: Behältnis
- 3: Aufnahmeeinheit
- 4: Gehäuse
- 5: Öffnungsklappe
- 6: Scharnier
- 7: Auffangschale
- 8: erste Halbschale
- 9: zweite Halbschale
- 10: Fixiermittel
- 11: Halbschalen-Basis
- 12: Halbschalen-Basis
- 13: Seitenwandabschnitt
- 14: Seitenwandabschnitt
- 15: Schichtaufbau
- 16: Heizelement
- 17: Außenhaut
- 18: elektrische Isolationsschicht
- 19: Träger- bzw. Isolationsschicht
- 20: Montageträger
- 21: erste Bewegungseinrichtung
- 22: zweite Bewegungseinrichtung
- 23: Bewegungspfeil
- 24: Bewegungspfeil
- 25: Linearantrieb
- 26: mechanische Koppeleinrichtung
- 27: Führungseinheit
- 28: Verbindungsmittel
- 29: Statoranordnung
- 30: Läuferanordnung
- 31: Führungsstange
- 32: Führungsstange
- 33: Federsystem
- 34: Leistungselektronik
- 35: Elektronik
- 36: zweite Bewegungseinheit
- 37: Bewegungspfeil
- 38: Bewegungspfeil
- 39: Kraftübertragungselement
- 40: Kugellagerung
- 41: Schweißverbindung
- 42: Hubmagnet
- 43: Gestänge
- 44: Schlitten
- 45: Führungsstange
- 46: Bewegungspfeil
- 47: mechanische Hubeinrichtung
- 48: Kraftübertragungsstift
- 49: Gehäuseteil
- 50: Schlitten
- 51: Führungsstange
- 52: Steuereinheit
- 53: Bewegungsmechanik
- 54: Getriebemotor
- 55: Nockenwelle
- 56: Stößel
- 57: Ausnehmung
- 58: Stößelplatte
- 59: Neodymium-Permanentmagnete
- 60: Cu-Flachspule
- 61: Geblechter Fe-Stator-Rückschluss
- 62: Geblechter Fe-Läufer-Rückschluss
- 63: Läuferlager
- 64: Trafo
- 65: Trafo
- 66: Joch
- 67: Joch
- 68: Trafo
- 69: Schlitten
- 70: Bewegungspfeil
- E: Ebene
- H: Hubachse
- L: Halbschalen-Längsachse

## Patentansprüche

1. Auftauvorrichtung zum Auftauen eines in gefrorenem Aggregatszustand befindlichen Substrats (1), insbesondere Blutplasma, wobei das Substrat (1) in einem Behältnis (2), insbesondere einem Beutel, enthalten ist, die Auftauvorrichtung umfassend
a. eine Aufnahmeeinheit (3) zur Anordnung des das Substrat (1) enthaltenden Behältnisses (2), wobei die Aufnahmeeinheit (3) eine Heizeinrichtung zur Bereitstellung von zum Auftauen des Substrats (1) benötigter Wärmeenergie aufweist;
b. eine erste Bewegungseinrichtung (21), die dazu eingerichtet ist, eine erste Bewegung auf die Aufnahmeeinheit (3) sowie das darin aufgenommene Behältnis (2) samt Substrat (1) zu übertragen, wobei die erste Bewegung eine im Wesentlichen in einer Horizontalebene ausgeführte Schüttelbewegung ist;
c. eine zweite Bewegungseinrichtung (22), die dazu eingerichtet ist, eine zweite Bewegung auf die Aufnahmeeinheit (3) sowie das darin aufgenommene Behältnis (2) samt Substrat (1) zu übertragen, wobei die zweite Bewegung eine Walkbewegung ist, wobei die zweite Bewegungseinrichtung (22) zumindest eine zweite Bewegungseinheit (36) umfasst, die dazu eingerichtet ist, eine vertikale Hubbewegung auf die Aufnahmeeinheit (3) auszuüben, wobei die Ausübung der Hubbewegung eine partielle Deformation der Aufnahmeeinheit (3) in einem Maß bewirkt, das zu einem aufgetauten Anteil des Substrats (1) korrespondiert.

2. Auftauvorrichtung nach Anspruch 1, **dadurch gekennzeichnet, dass** die Aufnahmeeinheit (3) in Form einer Schale ausgebildet ist, wobei die Schale eine erste Halbschale (8) und eine zweite Halbschale (9) umfasst, wobei die erste Halbschale (8) eine untere Halbschale und die zweite Halbschale (9) eine obere Halbschale ist, und wobei die erste und zweite Halbschale (8, 9) lösbar miteinander verbindbar sind, wobei die erste und zweite Halbschale (8, 9) vorzugsweise an die Form des Behältnisses (2) angepasst sind, wobei die erste und zweite Halbschale (8, 9) das Behältnis (2) bei dessen Anordnung in der Schale zumindest teilweise umgeben.

3. Auftauvorrichtung nach Anspruch 2, **dadurch gekennzeichnet, dass** an der ersten und zweiten Halbschalte (8, 9) Fixiermittel (10) vorgesehen sind, mit denen das Behältnis (2) fixierbar ist.

4. Auftauvorrichtung nach Anspruch 2 oder Anspruch 3, **dadurch gekennzeichnet, dass** die erste und zweite Halbschale (8, 9) jeweils einen mehrlagigen Schichtaufbau (15) aufweist, wobei jeweils in einer Schicht des Schichtaufbaus (15) einer jeweiligen Halbschale (8, 9) ein Heizelement (16) vorgesehen ist.

5. Auftauvorrichtung nach einem der vorangehenden Ansprüche, **dadurch gekennzeichnet, dass** die erste Bewegungseinrichtung (21) einen Linearantrieb (25) umfasst, der über eine mechanische Koppeleinrichtung (26) mit zumindest einer beweglich gelagerten Führungseinheit (27) in mechanischer Wirkverbindung steht, wobei die zumindest eine Führungseinheit (27) zur Übertragung der ersten Bewegung mit der Aufnahmeeinheit (3) mechanisch wirkverbunden ist, wobei die mechanische Koppeleinrichtung (26) vorzugsweise ein Kreuzfedergelenk ist, welches zwischen dem Linearantrieb (25) und der zumindest einen Führungseinheit (27) angeordnet und sowohl mit dem Linearantrieb (25) als auch mit der zumindest einen Führungseinheit (27) oder einem damit wirkverbundenen Bauteil über ein Verbindungsmittel (28), beispielsweise ein Edelstahlband, verbunden ist.

6. Auftauvorrichtung nach Anspruch 5, **dadurch gekennzeichnet, dass** der Linearantrieb (25) mit einem mechanischen oder elektrischen Federsystem (33) verbunden ist, welches dazu eingerichtet ist, Laufeigenschaften und Wirkungsgrad des Linearantriebs (25) einzustellen.

7. Auftauvorrichtung nach Anspruch 5 oder Anspruch 6, **dadurch gekennzeichnet, dass** der Linearantrieb (25) ein elektromechanischer Linearantrieb ist, welcher zumindest eine Stator- und eine Läuferanordnung (29, 30) aufweist, wobei die Läuferanordnung (30) über das Verbindungsmittel (28), insbesondere das Edelstahlband, mit der mechanischen Koppeleinrichtung (26) verbunden ist.

8. Auftauvorrichtung nach Anspruch 7, **dadurch gekennzeichnet, dass** die zumindest eine Führungseinheit (27) und die Läuferanordnung (30) jeweils auf Führungsstangen (31, 32) beweglich gelagert sind, wobei die Führungsstangen (31, 32) vorzugsweise in einer gemeinsamen Ebene (E) angeordnet sind.

9. Auftauvorrichtung nach einem der Ansprüche 2 bis 4 oder einem der Ansprüche 5 bis 8 in Verbindung mit einem der Ansprüche 2 bis 4, **dadurch gekennzeichnet, dass** die zweite Bewegungseinrichtung (22) zumindest eine zweite Bewegungseinheit (36) umfasst, die dazu eingerichtet ist, eine Hubbewegung auf die untere Halbschale (8) der Aufnahmeeinheit (3) auszuüben, wobei bei Ausübung der Hubbewegung eine partielle Deformation der unteren Halbschale (8) der Aufnahmeeinheit (3) in einem Maß bewirkt, das zu einem aufgetauten Anteil des Substrats (1) korrespondiert.

10. Auftauvorrichtung nach einem der Ansprüche 2 bis 4 oder einem der Ansprüche 5 bis 8 in Verbindung mit einem der Ansprüche 2 bis 4, **dadurch gekennzeichnet, dass** zwischen der Aufnahmeeinheit (3), insbesondere der unteren Halbschale (8), und der zumindest einen zweiten Bewegungseinheit (36) ein Kraftübertragungselement (39) angeordnet ist, welches zur Übertragung der Hubbewegung auf die Aufnahmeeinheit (3), insbesondere die untere Halbschale (8), sowohl mit der Aufnahmeeinheit (3), insbesondere der unteren Halbschale (8), als auch mit der zumindest einen zweiten Bewegungseinheit (36) verbunden ist.

11. Auftauvorrichtung nach Anspruch 10, **dadurch gekennzeichnet, dass** das Kraftübertragungselement (39) eine Kugellagerung (40) umfasst, und dass das Kraftübertragungselement (39) über eine Schweißverbindung (41) an der Aufnahmeeinheit (3), insbesondere der unteren Halbschale (8), befestigt ist.

12. Auftauvorrichtung nach Anspruch 10 oder Anspruch 11, **dadurch gekennzeichnet, dass** die zumindest eine zweite Bewegungseinheit (36) zumindest einen Hubmagneten (42) umfasst, welcher dazu eingerichtet ist, durch Ausführen von Hubbewegungen einen Hub, insbesondere einen Hebe- und Senkhub, auf das Kraftübertragungselement (39), die mit dem Kraftübertragungselement (39) verbundene Aufnahmeeinheit (3) und das in der Aufnahmeeinheit (3) angeordnete Behältnis (2) samt darin enthaltenem Substrat (1) zu übertragen.

13. Auftauvorrichtung nach einem der Ansprüche 10 bis 12, **dadurch gekennzeichnet, dass** die zumindest eine zweite Bewegungseinheit (36) zumindest eine mechanische Hubeinrichtung (47) umfasst, die dazu eingerichtet, durch Ausführen von Hubbewegungen einen Hub, insbesondere einen Hebe- und Senkhub, auf einen quer, insbesondere senkrecht, zur Hubachse (H) bewegbar angeordneten Kraftübertragungsstift (48) zu übertragen.

14. Auftauvorrichtung nach Anspruch 13, **dadurch gekennzeichnet, dass** der Kraftübertragungsstift (48) quer, insbesondere senkrecht, zur Hubachse (H) zwischen einer ersten und einer zweiten Stellung (101, 102) bewegbar ist, wobei der Kraftübertragungsstift (48) dazu eingerichtet ist, in seiner zweiten Stellung (102) eine von der zumindest einen mechanischen Hubeinrichtung (47) ausgeführte Hubbewegung auf das Kraftübertragungselement (39), die mit dem Kraftübertragungselement (39) verbundene Aufnahmeeinheit (3) und das in der Aufnahmeeinheit (3) angeordnete Behältnis (2) samt darin enthaltenem Substrat (1) zu übertragen.

15. Verfahren zum Auftauen eines in gefrorenem Aggregatszustand befindlichen Substrats (1), insbesondere Blutplasma, wobei das Substrat (1) in einem Behältnis (2), insbesondere einem Beutel, enthalten ist, mit einer Auftauvorrichtung gemäß einem der Ansprüche 1 bis 14, umfassend die folgenden Schritte:
a. Anordnen des das Substrat (1) enthaltenden Behältnisses (2) in der Aufnahmeeinheit (3),
b. Aufheizen des in dem Behältnis (2) enthaltenen Substrats (1) mit der Heizeinrichtung, wobei während des Aufheizens
i. mit der ersten Bewegungseinrichtung (21) eine erste Bewegung auf die Aufnahmeeinheit (3) sowie das darin aufgenommene Behältnis (2) samt Substrat (1) übertragen wird, wobei die erste Bewegung eine im Wesentlichen in einer Horizontalebene ausgeführte Schüttelbewegung, ist, sowie
ii. mit der zweiten Bewegungseinrichtung (22) eine zweite Bewegung auf die Aufnahmeeinheit (3) sowie das darin aufgenommene Behältnis (2) samt Substrat (1) übertragen wird, wobei die zweite Bewegung eine Walkbewegung ist, wobei die zweite Bewegungseinrichtung (22) zumindest eine zweite Bewegungseinheit (36) umfasst, die dazu eingerichtet ist, eine vertikale Hubbewegung auf die Aufnahmeeinheit (3) auszuüben, wobei die Ausübung der Hubbewegung eine partielle Deformation der Aufnahmeeinheit (3) in einem Maß bewirkt, das zu einem aufgetauten Anteil des Substrats (1) korrespondiert.

## Claims

1. Thawing device for thawing a substrate (1) in a frozen aggregate state, in particular blood plasma, the substrate (1) being contained in a container (2), in particular a bag, the thawing device comprising:
a. a receiving unit (3) for arranging the container (2) containing the substrate (1), the receiving unit (3) having a heating device for providing thermal energy required for thawing the substrate (1);
b. a first movement device (21), which is set up to transmit a first movement to the receiving unit (3) as well as the container (2) received therein together with the substrate (1), wherein the first movement is a shaking movement executed essentially in a horizontal plane;
c. a second movement device (22), which is set up to transmit a second movement to the receiving unit (3) as well as the container (2) received therein together with the substrate (1), wherein the second movement is a fulling movement, wherein the second movement device (22) comprises at least one second movement unit (36), which is set up to exert a vertical lifting movement on the receiving unit (3), wherein a partial deformation of the receiving unit (3) is effected to an extent corresponding to a thawed portion of the substrate (1) when the lifting movement is exerted.

2. Thawing device according to claim 1, **characterized in that** the receiving unit (3) is formed in the shape of a shell, wherein the shell comprises a first half shell (8) and a second half shell (9), wherein the first half shell (8) is a lower half shell and the second half shell (9) is an upper half shell, and wherein the first and second half shells (8, 9) are detachably connectable to each other, wherein the first and second half-shells (8, 9) are preferably adapted to the shape of the container (2), the first and second half-shells (8, 9) at least partially surrounding the container (2) when the container is placed in the shell.

3. Thawing device according to claim 2, **characterized in that** fixing means (10) are provided on the first and second half-shells (8, 9), with which the container (2) can be fixed.

4. Thawing device according to claim 2 or claim 3, **characterized in that** the first and second half shells (8, 9) each have a multilayer layer structure (15), a heating element (16) being provided in each case in one layer of the layer structure (15) of a respective half shell (8, 9).

5. Thawing device according to one of the preceding claims, **characterized in that** the first movement device (21) comprises a linear drive (25) which is in mechanical operative connection with at least one movably mounted guide unit (27) via a mechanical coupling device (26), the at least one guide unit (27) being mechanically operatively connected to the receiving unit (3) for transmitting the first movement wherein the mechanical coupling device (26) is preferably a universal spring joint which is arranged between the linear drive (25) and the at least one guide unit (27) and is connected both to the linear drive (25) and to the at least one guide unit (27) or to a component operatively connected thereto via a connecting means (28), for example a stainless steel strip.

6. Thawing device according to claim 5, **characterized in that** the linear drive (25) is connected to a mechanical or electrical spring system (33), which is adapted to adjust running characteristics and efficiency of the linear drive (25).

7. Thawing device according claim 5 or claim 6, **characterized in that** the linear drive (25) is an electromechanical linear drive, which has at least one stator and one rotor arrangement (29, 30), the rotor arrangement (30) being connected to the mechanical coupling device (26) via the connecting means (28), in particular the stainless steel strip.

8. Thawing device according claim 7, **characterized in that** the at least one guide unit (27) and the rotor arrangement (30) are each movably mounted on guide rods (31, 32), the guide rods (31, 32) preferably being arranged in a common plane (E).

9. Thawing device according to one of the claims 2 to 4, **characterized in that** the second movement device (22) comprises at least one second movement unit (36), which is arranged to exert a lifting movement on the lower half-shell (8) of the receiving unit (3), wherein, when the lifting movement is exerted, a partial deformation of the lower half-shell (8) of the receiving unit (3) is effected to an extent corresponding to a thawed portion of the substrate (1).

10. Thawing device according to one of claims 2 to 4, **characterized in that** a force transmission element (39) is arranged between the receiving unit (3), in particular the lower half shell (8), and the at least one second movement unit (36), which force transmission element (39) is connected both to the receiving unit (3), in particular the lower half shell (8), and to the at least one second movement unit (36) in order to transmit the lifting movement to the receiving unit (3), in particular the lower half shell (8).

11. Thawing device according to claim 10, **characterized in that** the force transmission element (39) comprises a ball bearing (40), and **in that** the force transmission element (39) is fastened to the receiving unit (3), in particular the lower half shell (8), via a welded joint (41).

12. Thawing device according to claim 10 or claim 11, **characterized in that** the at least one second movement unit (36) comprises at least one lifting magnet (42), which is set up to transmit a stroke, in particular a lifting and lowering stroke, to the force transmission element (39), the receiving unit (3) connected to the force transmission element (39) and the container (2) arranged in the receiving unit (3), together with the substrate (1) contained therein, by carrying out lifting movements.

13. Thawing device according to one of claims 10 to 12, **characterized in that** the at least one second movement unit (36) comprises at least one mechanical lifting device (47), which is set up to transmit a stroke, in particular a lifting and lowering stroke, to a force transmission pin (48) arranged movably transversely, in particular perpendicularly, to the stroke axis (H) by carrying out lifting movements.

14. Thawing device according to claim 13, **characterized in that** the force transmission pin (48) can be moved transversely, in particular perpendicularly, to the stroke axis (H) between a first and a second position (101, 102), the force transmission pin (48) being arranged to in its second position (102), to transmit a lifting movement executed by the at least one mechanical lifting device (47) to the force-transmitting element (39), the receiving unit (3) connected to the force-transmitting element (39) and the container (2) arranged in the receiving unit (3), together with the substrate (1) contained therein.

15. Method for thawing a substrate (1) in a frozen aggregate state, in particular blood plasma, wherein the substrate (1) is contained in a container (2), in particular a bag, with a thawing device according to one of claims 1 to 14, comprising the following steps:
a. Arrangement of the container (2) containing the substrate (1) in the receiving unit (3),
b. Heating the substrate (1) contained in the container (2) with the heating device, wherein during the heating process
i. a first movement is transmitted by the first movement device (21) to the receiving unit (3) and the container (2) received therein together with the substrate (1), the first movement being a shaking movement executed substantially in a horizontal plane, and
ii. a second movement is transmitted by the second movement device (22) to the receiving unit (3) and the container (2) received therein together with the substrate (1), the second movement being a fulling movement, wherein the second movement device (22) comprises at least one second movement unit (36), which is set up to exert a vertical lifting movement on the receiving unit (3), wherein a partial deformation of the receiving unit (3) is effected to an extent corresponding to a thawed portion of the substrate (1) when the lifting movement is exerted.

## Revendications

1. Dispositif de décongélation permettant de décongeler un substrat (1) dans un état agrégé gelé, en particulier du plasma sanguin, le substrat (1) étant contenu dans un conteneur (2), en particulier un sac, le dispositif de décongélation comprenant :
a. une unité de réception (3) pour disposer le conteneur (2) contenant le substrat (1), l'unité de réception (3) ayant un dispositif de chauffage pour fournir l'énergie thermique requise pour décongeler le substrat (1) ;
b. un premier dispositif de mouvement (21), qui est conçu pour transmettre un premier mouvement à l'unité de réception (3) ainsi qu'au conteneur (2) reçu dans celle-ci conjointement avec le substrat (1), dans lequel le premier mouvement est un mouvement de secousse exécuté essentiellement dans un plan horizontal ;
c. un deuxième dispositif de mouvement (22), qui est conçu pour transmettre un deuxième mouvement à l'unité de réception (3) ainsi qu'au conteneur (2) reçu dans celle-ci conjointement avec le substrat (1), dans lequel le deuxième mouvement est un mouvement de foulage, dans lequel le deuxième dispositif de mouvement (22) comprend au moins une deuxième unité de mouvement (36), qui est conçue pour exercer un mouvement de levage vertical sur l'unité de réception (3), dans lequel une déformation partielle de l'unité de réception (3) est effectuée dans une mesure correspondant à une partie décongelée du substrat (1) lorsque le mouvement de levage est exercé.

2. Dispositif de décongélation selon la revendication 1, **caractérisé en ce que** l'unité de réception (3) est en forme de coque, dans lequel la coque comprend une première demi-coque (8) et une deuxième demi-coque (9), dans lequel la première demi-coque (8) est une demi-coque inférieure et la deuxième demi-coque (9) est une demi-coque supérieure, et dans lequel les première et deuxième demi-coques (8, 9) sont adaptées pour être reliées de manière amovible les unes aux autres, dans lequel les première et deuxième demi-coques (8, 9) sont adaptées de préférence à la forme du conteneur (2), les première et deuxième demi-coques (8, 9) entourant au moins partiellement le conteneur (2) lorsque le conteneur est placé dans la coque.

3. Dispositif de décongélation selon la revendication 2, **caractérisé en ce que** des moyens de fixation (10) sont fournis sur les première et deuxième demi-coques (8, 9), grâce auxquels le conteneur (2) peut être fixé.

4. Dispositif de décongélation selon la revendication 2 ou la revendication 3, **caractérisé en ce que** les première et deuxième demi-coques (8, 9) ont chacune une structure de couches multicouches (15), un élément de chauffage (16) étant fourni dans chaque cas dans une couche de la structure de couches (15) d'une demi-coque respective (8, 9).

5. Dispositif de décongélation selon l'une des revendications précédentes, **caractérisé en ce que** le premier dispositif de mouvement (21) comprend un entraînement linéaire (25) qui est en liaison fonctionnelle mécanique avec au moins une unité de guidage montée de manière mobile (27) par l'intermédiaire d'un dispositif de couplage mécanique (26), l'au moins une unité de guidage (27) étant reliée de manière fonctionnelle mécaniquement à l'unité de réception (3) pour transmettre le premier mouvement dans lequel le dispositif de couplage mécanique (26) est de préférence un joint à ressort universel qui est disposé entre l'entraînement linéaire (25) et l'au moins une unité de guidage (27) et est relié à la fois à l'entraînement linéaire (25) et à l'au moins une unité de guidage (27) ou à un composant relié de manière fonctionnelle à celui-ci par l'intermédiaire des moyens de liaison (28), par exemple une bande d'acier inoxydable.

6. Dispositif de décongélation selon la revendication 5, **caractérisé en ce que** l'entraînement linéaire (25) est relié à un système de ressort électrique ou mécanique (33), qui est adapté pour régler les caractéristiques de fonctionnement et d'efficacité de l'entraînement linéaire (25).

7. Dispositif de décongélation selon la revendication 5 ou la revendication 6, **caractérisé en ce que** l'entraînement linéaire (25) est un entraînement linéaire électromécanique, qui a au moins un stator et un agencement de rotors (29, 30), l'agencement de rotors (30) étant relié au dispositif de couplage mécanique (26) par l'intermédiaire des moyens de liaison (28), en particulier la bande d'acier inoxydable.

8. Dispositif de décongélation selon la revendication 7, **caractérisé en ce que** l'au moins une unité de guidage (27) et l'agencement de rotors (30) sont chacun montés de manière mobile sur des tiges de guidage (31, 32), les tiges de guidage (31, 32) étant de préférence disposées dans un plan commun (E).

9. Dispositif de décongélation selon l'une des revendications 2 à 4, **caractérisé en ce que** le deuxième dispositif de mouvement (22) comprend au moins une deuxième unité de mouvement (36), qui est disposée pour exercer un mouvement de levage sur la demi-coque inférieure (8) de l'unité de réception (3), dans lequel, lorsque le mouvement de levage est exercé, une déformation partielle de la demi-coque inférieure (8) de l'unité de réception (3) est effectuée dans une mesure correspondant à une partie décongelée du substrat (1).

10. Dispositif de décongélation selon l'une des revendications 2 à 4, **caractérisé en ce qu'**un élément de transmission de force (39) est disposé entre l'unité de réception (3), en particulier la demi-coque inférieure (8), et l'au moins une deuxième unité de mouvement (36), ledit élément de transmission de force (39) est relié à la fois à l'unité de réception (3), en particulier la demi-coque inférieure (8), et à l'au moins une deuxième unité de mouvement (36) afin de transmettre le mouvement de levage à l'unité de réception (3), en particulier la demi-coque inférieure (8).

11. Dispositif de décongélation selon la revendication 10, **caractérisé en ce que** l'élément de transmission de force (39) comprend un roulement à billes (40), et **en ce que** l'élément de transmission de force (39) est fixé à l'unité de réception (3), en particulier la demi-coque inférieure (8), par l'intermédiaire d'un joint soudé (41).

12. Dispositif de décongélation selon la revendication 10 ou la revendication 11, **caractérisé en ce que** l'au moins une deuxième unité de mouvement (36) comprend au moins un aimant de levage (42), qui est conçu pour transmettre une course, en particulier une course de levage et d'abaissement, à l'élément de transmission de force (39), l'unité de réception (3) reliée à l'élément de transmission de force (39) et le conteneur (2) disposé dans l'unité de réception (3), conjointement avec le substrat (1) contenu dans celle-ci, par la mise en oeuvre de mouvements de levage.

13. Dispositif de décongélation selon l'une des revendications 10 à 12, **caractérisé en ce que** l'au moins une deuxième unité de mouvement (36) comprend au moins un dispositif de levage mécanique (47), qui est conçu pour transmettre une course, en particulier une course de levage et d'abaissement, à une broche de transmission de force (48) disposée de manière mobile et transversale, en particulier perpendiculairement, à l'axe de course (X) par la mise en oeuvre des mouvements de levage.

14. Dispositif de décongélation selon la revendication 13, **caractérisé en ce que** la broche de transmission de force (48) peut être déplacée de manière transversale, en particulier perpendiculairement, à l'axe de course (H) entre une première et une deuxième position (101, 102), la broche de transmission de force (48) étant disposée dans sa deuxième position (102), pour transmettre un mouvement de levage exécuté par l'au moins un dispositif de levage mécanique (47) à l'élément de transmission de force (39), l'unité de réception (3) reliée à l'élément de transmission de force (39) et le conteneur (2) disposé dans l'unité de réception (3), conjointement avec le substrat (1) contenu dans celle-ci.

15. Procédé de décongélation d'un substrat (1) dans un état agrégé gelé, en particulier du plasma sanguin, dans lequel le substrat (1) est contenu dans un conteneur (2), en particulier un sac, avec un dispositif de décongélation selon l'une des revendications 1 à 14, comprenant les étapes suivantes :
a. disposition du conteneur (2) contenant le substrat (1) dans l'unité de réception (3),
b. chauffage du substrat (1) contenu dans le conteneur (2) avec le dispositif de chauffage, dans lequel pendant le processus de chauffage
i. un premier mouvement est transmis par le premier dispositif de mouvement (21) à l'unité de réception (3) et au conteneur (2) reçu dans celle-ci conjointement avec le substrat (1), le premier mouvement étant un mouvement de secousse exécuté sensiblement dans un plan horizontal, et
ii. un deuxième mouvement est transmis par le deuxième dispositif de mouvement (22) à l'unité de réception (3) et au conteneur (2) reçu dans celle-ci conjointement avec le substrat (1), le deuxième mouvement étant un mouvement de foulage, dans lequel le deuxième dispositif de mouvement (22) comprend au moins une deuxième unité de mouvement (36), qui est conçue pour exercer un mouvement de levage vertical sur l'unité de réception (3), dans lequel une déformation partielle de l'unité de réception (3) est effectuée dans une mesure correspondant à une partie décongelée du substrat (1) lorsque le mouvement de levage est exercé.
